# EUROPEAN PATENT SPECIFICATION

(11) **EP 2 262 799 B1**
(45) Date of publication and mention of the grant of the patent: **04.07.2012**
(21) Application number: 09718458.4
(22) Date of filing: 03.03.2009
(51) Int. Cl.: C07D 413/04, A61K 31/4427, A61P 37/00

(54) **PYRIDINE COMPOUNDS**
PYRIDINVERBINDUNGEN
COMPOSÉS DE PYRIDINE

(30) Priority: 06.03.2008 WO PCT/IB2008/050819
(43) Date of publication of application: 22.12.2010
(73) Proprietor: Actelion Pharmaceuticals Ltd., 4123 Allschwil (CH)
(72) Inventor: BOLLI, Martin, CH-4123 Allschwil (CH); LESCOP, Cyrille, F-68680 Kembs (FR); MATHYS, Boris, CH-4133 Pratteln (CH); MUELLER, Claus, 79576 Weil Am Rhein (DE); NAYLER, Oliver, CH-4144 Arlesheim (CH); STEINER, Beat, CH-4143 Dornach (CH)
(74) Representative: Gschwend, Thomas Peter
(86) International application number: PCT/IB2009/050847
(87) International publication number: WO 2009/109906

(56) References cited:
- WO-A-2005/032465
- WO-A-2005/058848
- WO-A-2007/085451
- WO-A-2007/132307

## Description

### Field of the invention

The present invention relates to S1P1/EDG1 receptor agonists of Formula (I) and their use as active ingredients in the preparation of pharmaceutical compositions. The invention also concerns related aspects including processes for the preparation of the compounds, pharmaceutical compositions containing a compound of the Formula (I), and their use as compounds improving vascular function and as immunomodulating agents, either alone or in combination with other active compounds or therapies.

### Background of the invention

The human immune system is designed to defend the body against foreign micro-organisms and substances that cause infection or disease. Complex regulatory mechanisms ensure that the immune response is targeted against the intruding substance or organism and not against the host. In some cases, these control mechanisms are unregulated and autoimmune responses can develop. A consequence of the uncontrolled inflammatory response is severe organ, cell, tissue or joint damage. With current treatment, the whole immune system is usually suppressed and the body's ability to react to infections is also severely compromised. Typical drugs in this class include azathioprine, chlorambucil, cyclophosphamide, cyclosporin, or methotrexate. Corticosteroids which reduce inflammation and suppress the immune response, may cause side effects when used in long term treatment. Nonsteroidal anti-infammatory drugs (NSAIDs) can reduce pain and inflammation, however, they exhibit considerable side effects. Alternative treatments include agents that activate or block cytokine signaling.

Orally active compounds with immunomodulating properties, without compromising immune responses and with reduced side effects would significantly improve current treatments of uncontrolled inflammatory disease.

In the field of organ transplantation the host immune response must be suppressed to prevent organ rejection. Organ transplant recipients can experience some rejection even when they are taking immunosuppressive drugs. Rejection occurs most frequently in the first few weeks after transplantation, but rejection episodes can also happen months or even years after transplantation. Combinations of up to three or four medications are commonly used to give maximum protection against rejection while minimizing side effects. Current standard drugs used to treat the rejection of transplanted organs interfere with discrete intracellular pathways in the activation of T-type or B-type white blood cells. Examples of such drugs are cyclosporin, daclizumab, basiliximab, everolimus, or FK506, which interfere with cytokine release or signaling; azathioprine or leflunomide, which inhibit nucleotide synthesis; or 15-deoxyspergualin, an inhibitor of leukocyte differentiation.

The beneficial effects of broad immunosuppressive therapies relate to their effects; however, the generalized immunosuppression which these drugs produce diminishes the immune system's defense against infection and malignancies. Furthermore, standard immunosuppressive drugs are often used at high dosages and can cause or accelerate organ damage.

### Description of the invention

The present invention provides novel compounds of Formula (I) that are agonists for the G protein-coupled receptor S1P1/EDG1 and have a powerful and long-lasting immunomodulating effect which is achieved by reducing the number of circulating and infiltrating T- and B-lymphocytes, without affecting their maturation, memory, or expansion. The reduction of circulating T- / B-lymphocytes as a result of S1P1/EDG1 agonism, possibly in combination with the observed improvement of endothelial cell layer function associated with S1P1/EDG1 activation, makes such compounds useful to treat uncontrolled inflammatory disease and to improve vascular functionality.

The compounds of the present invention can be utilized alone or in combination with standard drugs inhibiting T-cell activation, to provide a new immunomodulating therapy with a reduced propensity for infections when compared to standard immunosuppressive therapy. Furthermore, the compounds of the present invention can be used in combination with reduced dosages of traditional immunosuppressant therapies, to provide on the one hand effective immunomodulating activity, while on the other hand reducing end organ damage associated with higher doses of standard immunosuppressive drugs. The observation of improved endothelial cell layer function associated with S1P1/EDG1 activation provides additional benefits of compounds to improve vascular function.

The nucleotide sequence and the amino acid sequence for the human S1P1/EDG1 receptor are known in the art and are published in e.g.: Hla, T., and Maciag, T. J. Biol Chem. 265 (1990), 9308-9313; WO 91/15583 published 17 October 1991; WO 99/46277 published 16 September 1999. The potency and efficacy of the compounds of Formula (I) are assessed using a GTPγS assay to determine EC₅₀ values and by measuring the circulating lymphocytes in the rat after oral administration, respectively (see in Examples).
i) The invention relates to novel pyridine compounds of Formula (I), 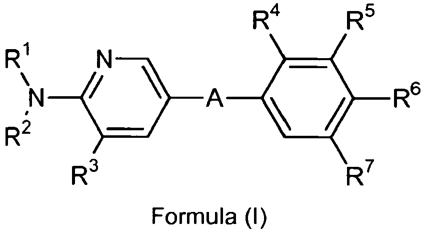 wherein
   **A** represents 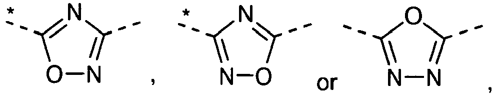 wherein the asterisks indicate the bond that is linked to the pyridine group of Formula (I);
   **R¹** represents C₁₋₄-alkyl;
   **R²** represents hydrogen or C₁₋₃-alkyl;
   or **R¹** and **R²**, together with the nitrogen to which they are attached, form an azetidine, or a pyrrolidine ring;
   **R³** represents C₁₋₃-alkyl;
   **R⁴** represents hydrogen, methoxy, or methyl (notably hydrogen, or methoxy);
   **R⁵** represents hydrogen, C₁₋₃-alkyl, or methoxy;
   **R⁶** represents -CH₂-(CH₂)ₙ-NR⁶¹R⁶², -CH₂-CH₂-CONR⁶¹R⁶², 1-(3-carboxy-azetidinyl)-3-propionyl, 1-(2-carboxy-pyrrolidinyl)3-propionyl, 1-(3-carboxy-pyrrolidinyl)-3-propionyl, hydroxy, hydroxy-C₂₋₄-alkoxy, di-(hydroxy-C₁₋₂-alkyl)-C₁₋₂-alkoxy, 2,3-dihydroxypropoxy, -OCH₂-(CH₂)ₙ-NR⁶¹R⁶², -OCH₂-(CH₂)ₙ-NHCOR⁶³, 2-[(azetidine-3-carboxylic acid)-1-yl]-ethoxy, 2-[(pyrrolidine-2-carboxylic acid)-1-yl]-ethoxy, 2-[(pyrrolidine-3-carboxylic acid)-1-yl]-ethoxy, -OCH₂-CH(OH)-CH₂-NR⁶¹R⁶², -OCH₂-CH(OH)-CH₂-NHCOR⁶³, 3-[(azetidine-3-carboxylic acid)-1-yl]-2-hydroxypropoxy, 2-hydroxy-3-[(pyrrolidine-2-carboxylic acid)-1-yl]-propoxy, or 2-hydroxy-3-[(pyrrolidine-3-carboxylic acid)-1-yl]-propoxy;
   **R⁶¹** represents hydrogen, methyl, 2-hydroxyethyl, 2-aminoethyl, 2-(C₁₋₄-alkylamino)ethyl, 2-(di-(C₁₋₄-alkyl)amino)ethyl, carboxymethyl, (C₁₋₄-alkylcarboxy)methyl, 2-carboxyethyl, or 2-(C₁₋₄-alkylcarboxy)ethyl;
   **R⁶²** represents hydrogen or methyl;
   **R⁶³** represents hydroxymethyl, 2-hydroxyethyl, aminomethyl, methylaminomethyl, dimethylaminomethyl, or 2-methylamino-ethyl;
   n represents the integer 1, or 2; and
   **R⁷** represents hydrogen, methyl, or chloro.
   A particular sub-embodiment consists of compounds of Formula (I) according to embodiment i), wherein **R⁴** represents hydrogen or methoxy.
   Another particular sub-embodiment consists of compounds of Formula (I) according to embodiment i) wherein **R⁴** represents methyl.
   The general terms used hereinbefore and hereinafter preferably have, within this disclosure, the following meanings, unless otherwise indicated:
   The term "C_{x-y}-alkyl" (x and y each being an integer) refers to a saturated straight or branched hydrocarbon chain with x to y carbon atoms. For example, a C₁₋₄-alkyl group contains from one to four carbon atoms. Representative examples of C₁₋₄-alkyl groups include methyl, ethyl, *n*-propyl, *iso*-propyl, *n*-butyl, *iso*-butyl, *sec*-butyl, *tert*-butyl, Preferred examples of C₁₋₄-alkyl groups are methyl, ethyl, *n*-propyl, *iso*-propyl, *n*-butyl, and *iso*-butyl. Preferred examples of C₁₋₃-alkyl groups are methyl and ethyl.
   The term "C_{x-y}-alkoxy" (x and y each being an integer) refers to an alkyl-O- group wherein the alkyl group refers to a straight or branched hydrocarbon chain with x to y carbon atoms. For example, a C₂₋₄-alkoxy group contains two to four carbon atoms and includes ethoxy, *n-*propoxy, *iso*-propoxy, *n*-butoxy, *iso*-butoxy, *sec*-butoxy, and *tert*-butoxy. Preferred are ethoxy, *n*-propxy, and *iso*-propoxy. C₁₋₂-alkoxy groups refer to methoxy and ethoxy. Examples of "hydroxy-C₂₋₄-alkoxy" groups are 2-hydroxy-ethoxy, and 3-hydroxy-propoxy. An example of a " di-(hydroxy-C₁₋₂-alkyl)-C₁₋₂-alkoxy" group is di(hydroxymethyl)-methoxy.
ii) A second embodiment of the invention relates to compounds of Formula (I) according to embodiment i), wherein **A** represents 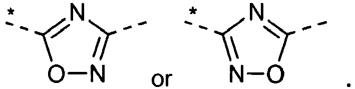
iii) Another embodiment of the invention relates to compounds of Formula (I) according to embodiment i), wherein A represents 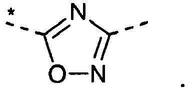
iv) Another embodiment of the invention relates to compounds of Formula (I) according to any one of the embodiments i) to iii), wherein **R¹** represents C₁₋₃-alkyl.
v) Another embodiment of the invention relates to compounds of Formula (I) according to any one of the embodiments i) to iii), wherein **R¹** represents C₂₋₃-alkyl.
vi) Another embodiment of the invention relates to compounds of Formula (I) according to any one of the embodiments i) to v), wherein **R²** represents methyl or ethyl.
vii) Another embodiment of the invention relates to compounds of Formula (I) according to any one of the embodiments i) to vi), wherein **R³** represents methyl or ethyl.
viii) Another embodiment of the invention relates to compounds of Formula (I) according to any one of the embodiments i) to vi), wherein **R³** represents methyl.
ix) Another embodiment of the invention relates to compounds of Formula (I) according to any one of the embodiments i) to viii), wherein **R⁴** represents hydrogen.
x) Another embodiment of the invention relates to compounds of Formula (I) according to any one of the embodiments i) to ix), wherein **R⁴** represents hydrogen, **R⁵** represents C₁₋₃-alkyl or methoxy, and **R⁷** represents methyl or chloro.
xi) Another embodiment of the invention relates to compounds of Formula (I) according to embodiments ix) or x), wherein **R⁵** represents C₁₋₂-alkyl or methoxy.
xii) Another embodiment of the invention relates to compounds of Formula (I) according to embodiments ix) or x), wherein **R⁵** represents ethyl or methoxy.
xiii) Another embodiment of the invention relates to compounds of Formula (I) according to embodiments ix) or x), wherein **R⁵** represents ethyl, and **R⁷** represents methyl.
xiv) Another embodiment of the invention relates to compounds of Formula (I) according to any one of the embodiments i) to xiii), wherein **R⁶** represents -CH₂-CH₂-CONR⁶¹R⁶², 1-(3-carboxy-azetidinyl)-3-propionyl, 1-(2-carboxy-pyrrolidinyl)-3-propionyl, 1-(3-carboxy-pyrrolidinyl)-3-propionyl, hydroxy-C₂₋₄-alkoxy, di-(hydroxy-C₁₋₂-alkyl)-C₁₋₂-alkoxy, 2,3-dihydroxypropoxy, -OCH₂-(CH₂)ₙ-NR⁶¹R⁶², -OCH₂-(CH₂)ₙ-NHCOR⁶³, 2-[(azetidine-3-carboxylic acid)-1-yl]-ethoxy, 2-[(pyrrolidine-2-carboxylic acid)-1-yl]-ethoxy, 2-[(pyrrolidine-3-carboxylic acid)-1-yl]-ethoxy, -OCH₂-CH(OH)-CH₂-NR⁶¹R⁶², -OCH₂-CH(OH)-CH₂-NHCOR⁶³, 3-[(azetidine-3-carboxylic acid)-1-yl]-2-hydroxypropoxy, 2-hydroxy-3-[(pyrrolidine-2-carboxylic acid)-1-yl]-propoxy, or 2-hydroxy-3-[(pyrrolidine-3-carboxylic acid)-1-yl]-propoxy.
xv) Another embodiment of the invention relates to compounds of Formula (I) according to any one of the embodiments i) to xiv), wherein **R⁶** represents -CH₂-CH₂-CONR⁶¹R⁶², 1-(3-carboxy-azetidinyl)-3-propionyl, 1-(2-carboxy-pyrrolidinyl)-3-propionyl, 1-(3-carboxy-pyrrolidinyl)-3-propionyl, hydroxy-C₂₋₄-alkoxy, di-(hydroxy-C₁₋₂-alkyl)-C₁₋₂-alkoxy, 2,3-dihydroxypropoxy, -OCH₂-(CH₂)ₙ-NHCOR⁶³, 2-[(azetidine-3-carboxylic acid)-1-yl]-ethoxy, -OCH₂-CH(OH)-CH₂NHCOR⁶³, or 3-[(azetidine-3-carboxylic acid)-1-yl]-2-hydroxypropoxy.
xvi) Another embodiment of the invention relates to compounds of Formula (I) according to any one of the embodiments i) to xv), wherein **R⁶** represents -CH₂-CH₂-CONR⁶¹R⁶², 2,3-dihydroxypropoxy, or -OCH₂-CH(OH)-CH₂-NHCOR⁶³.
xvii) Another embodiment of the invention relates to compounds of Formula (I) according to any one of the embodiments i) to xiv), wherein **R⁶** represents di-(hydroxy-C₁₋₂-alkyl)-C₁₋₂-alkoxy, 2,3-dihydroxypropoxy, -OCH₂CH(OH)-CH₂-NR⁶¹R⁶², -OCH₂-CH(OH)-CH₂-NHCOR⁶³, 3-[(azetidine-3-carboxylic acid)-1-yl]-2-hydroxypropoxy, 2-hydroxy-3-[(pyrrolidine-2-carboxylic acid)-1-yl]-propoxy, or 2-hydroxy-3-[(pyrrolidine-3-carboxylic acid)-1-yl]-propoxy.
xviii) Another embodiment of the invention relates to compounds of Formula (I) according to any one of the embodiments i) to xvii), wherein **R⁶** represents 2,3-dihydroxypropoxy, or -OCH₂-CH(OH)-CH₂-NHCOR⁶³.
xix) Another embodiment of the invention relates to compounds of Formula (I) according to any one of the embodiments i) to xviii), wherein **R⁶** represents -OCH₂CH(OH)-CH₂-NHCOR⁶³.
xx) Another particular embodiment of the invention relates to compounds of Formula (I) according to any one of the embodiments i) to xvii), wherein **R⁶¹** represents methyl, 2-hydroxyethyl, or carboxymethyl (especially 2-hydroxyethyl, or carboxymethyl).
xxi) Another embodiment of the invention relates to compounds of Formula (I) according to any one of the embodiments i) to xvii) or xx), wherein **R⁶²** represents hydrogen.
xxii) Another embodiment of the invention relates to compounds of Formula (I) according to any one of the embodiments i) to xxi), wherein **R⁶³** represents hydroxymethyl.
xxiii) Another embodiment of the invention relates to compounds of Formula (I) according to any one of the embodiments i) or iv) to xxi), wherein **A** represents 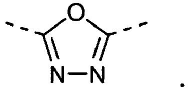
xxiv) A further embodiment of the invention relates to compounds of Formula (I) according to embodiment i), wherein at least one (preferably all) of the following characteristics are present:
   **A** represents 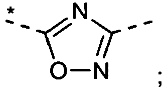
   **R¹** represents C₂₋₃-alkyl;
   **R²** represents C₁₋₂alkyl;
   or **R¹** and **R²**, together with the nitrogen to which they are attached, form a pyrrolidine ring;
   **R³** represents C₁₋₂-alkyl;
   **R⁴** represents hydrogen;
   **R⁵** represents C₁₋₂-alkyl;
   **R⁶** represents -CH₂-CH₂-CONR⁶¹R⁶², -OCH₂-CH(OH)-CH₂-NH₂, 1-(3-carboxy-azetidinyl)-3-propionyl, hydroxy, 2,3-dihydroxypropoxy, or -OCH₂-CH(OH)-CH₂-NHCOR⁶³;
   **R⁶¹** represents hydrogen, 2-hydroxyethyl, or carboxymethyl;
   **R⁶²** represents hydrogen;
   **R⁶³** represents hydroxymethyl;
   **R⁷** represents methyl.
xxv) A further embodiment of the invention relates to compounds of Formula (I) according to embodiment i), wherein at least one (preferably all) of the following characteristics are present:
   ● **A** represents 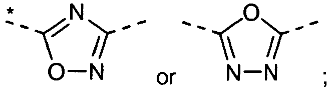
   ● **R¹** represents C₂₋₃-alkyl;
      **R²** represents C₁₋₂-alkyl;
      or **R¹** and **R²**, together with the nitrogen to which they are attached, form a pyrrolidine ring;
   ● **R³** represents C₁₋₂-alkyl;
   ● **R⁴** represents hydrogen; **R⁵** represents C₁₋₃-alkyl or methoxy; and **R⁷** represents methyl or chloro; or
      **R⁴** represents methyl or methoxy (notably methoxy); and **R⁵** and **R⁷** both represent hydrogen;
   ● **R⁶** represents -CH₂-CH₂-CONR⁶¹R⁶², -OCH₂-CH(OH)CH₂-NH₂, -OCH₂-CH(OH)-CH₂-NH-CH₃, -OCH₂-CH(OH)-CH₂-NH-CH₂-COOH, 1-(3-carboxy-azetidinyl)-3-propionyl, hydroxy, 2,3-dihydroxypropoxy, or -OCH₂-CH(OH)-CH₂-NHCOR⁶³;
   ● **R⁶¹** represents 2-hydroxyethyl, or carboxymethyl;
   ● **R⁶²** represents hydrogen;
   ● **R⁶³** represents hydroxymethyl.

The compounds of Formula (I) may contain one or more stereogenic or asymmetric centers, such as one or more asymmetric carbon atoms. The compounds of Formula (I) may thus be present as mixtures of stereoisomers or preferably as pure stereoisomers. Especially, compounds wherein **R⁶** represents a 2-hydroxy-propoxy derivative (such as for example 2,3-dihydroxypropoxy or -OCH₂-CH(OH)-CH₂-NHCOR⁶³) may be (R) or (S) configured. Mixtures of stereoisomers may be separated in a manner known to a person skilled in the art.

Where the plural form is used for compounds, salts, pharmaceutical compositions, diseases and the like, this is intended to mean also a single compound, salt, or the like.

Any reference hereinbefore or hereinafter to a compound of Formula (I) is to be understood as referring also to salts, especially pharmaceutically acceptable salts, of a compound of Formula (I), as appropriate and expedient.

Salts are preferably the pharmaceutically acceptable salts of the compounds of Formula (I).

The term "pharmaceutically acceptable salts" refers to non-toxic, inorganic or organic acid and/or base addition salts. Reference can be made to "Salt selection for basic drugs", Int. J. Pharm. (1986), 33, 201-217.

Examples of preferred compounds of Formula (I) according to embodiment i) are selected from the group consisting of:
N-[(R)-3-(2-Ethyl-4-{5-[6-(ethyl-methyl-amino)-5-methyl-pyridin-3-yl]-[1,2,4]oxadiazol-3-yl}-6-methyl-phenoxy)-2-hydroxy-propyl]-2-hydroxy-acetamide;
N-((S)-3-{4-[5-(6-Diethylamino-5-methyl-pyridin-3-yl)-[1,2,4]oxadiazol-3-yl]-2-ethyl-6-methyl-phenoxy}-2-hydroxy-propyl)-2-hydroxy-acetamide;
N-[(S)-3-(2-Ethyl-4-{5-[6-(isopropyl-methyl-amino)-5-methyl-pyridin-3-yl]-[1,2,4]oxadiazol-3-yl}-6-methyl-phenoxy)-2-hydroxy-propyl]-2-hydroxy-acetamide;
(S)-3-{4-[5-(6-Diethylamino-5-ethyl-pyridin-3-yl)-[1,2,4]oxadiazol-3-yl]-2-ethyl-6-methyl-phenoxy}-propane-1,2-diol;
N-((R)-3-{4-[5-(6-Diethylamino-5-ethyl-pyridin-3-yl)-[1,2,4]oxadiazol-3-yl]-2-ethyl-6-methyl-phenoxy}-2-hydroxy-propyl)-2-hydroxy-acetamide;
N-((S)-3-{4-[5-(6-Diethylamino-5-ethyl-pyridin-3-yl)-[1,2,4]oxadiazol-3-yl]-2-ethyl-6-methyl-phenoxy}-2-hydroxy-propyl)-2-hydroxy-acetamide;
(S)-3-(2-Ethyl-4-{5-[5-ethyl-6-(isopropyl-methyl-amino)-pyridin-3-yl]-[1,2,4]oxadiazol-3-yl}-6-methyl-phenoxy)-propane-1,2-diol;
N-[(R)-3-(2-Ethyl-4-{5-[5-ethyl-6-(isopropyl-methyl-amino)-pyridin-3-yl]-[1,2,4]oxadiazol-3-yl}-6-methyl-phenoxy)-2-hydroxy-propyl]-2-hydroxy-acetamide;
N-[(S)-3-(2-Ethyl-4-{5-[5-ethyl-6-(isopropyl-methyl-amino)-pyridin-3-yl]-[1,2,4]oxadiazol-3-yl}-6-methyl-phenoxy)-2-hydroxy-propyl]-2-hydroxy-acetamide;
(3-{4-[5-(6-Diethylamino-5-ethyl-pyridin-3-yl)-[1,2,4]oxadiazol-3-yl]-2-ethyl-6-methyl-phenyl}-propionylamino)-acetic acid;
3-(2-Ethyl-4-{5-[5-ethyl-6-(isopropyl-methyl-amino)-pyridin-3-yl]-[1,2,4]oxadiazol-3-yl}-6-methyl-phenyl)-N-(2-hydroxy-ethyl)-propionamide; and
[3-(2-Ethyl-4-{5-[5-ethyl-6-(isopropyl-methyl-amino)-pyridin-3-yl]-[1,2,4]oxadiazol-3-yl}-6-methyl-phenyl)-propionylamino]-acetic acid.

Further examples of preferred compounds of Formula (I) according to embodiment i) are selected from the group consisting of:
N-((S)-3-{2-Ethyl-4-[5-(5-ethyl-6-pyrrolidin-1-yl-pyridin-3-yl)-[1,2,4]oxadiazol-3-yl]-6-methyl-phenoxy}-2-hydroxy-propyl)-2-hydroxy-acetamide;
N-((S)-3-{4-[5-(6-Diethylamino-5-methyl-pyridin-3-yl)-[1,2,4]oxadiazol-3-yl]-2,6-dimethyl-phenoxy}-2-hydroxy-propyl)-2-hydroxy-acetamide;
N-((S)-3-{2-Chloro-4-[5-(6-diethylamino-5-methyl-pyridin-3-yl)-[1,2,4]oxadiazol-3-yl]-6-methyl-phenoxy}-2-hydroxy-propyl)-2-hydroxy-acetamide;
N-((S)-3-{4-[5-(6-Diethylamino-5-methyl-pyridin-3-yl)-[1,2,4]oxadiazol-3-yl]-2-methoxy-6-methyl-phenoxy}-2-hydroxy-propyl)-2-hydroxy-acetamide; N-((S)-3-{4-[5-(6-Diethylamino-5-methyl-pyridin-3-yl)-[1,2,4]oxadiazol-3-yl]-3-methyl-phenoxy}-2-hydroxy-propyl)-2-hydroxy-acetamide;
(S)-3-{2-Chloro-4-[5-(6-diethylamino-5-methyl-pyridin-3-yl)-[1,2,4]oxadiazol-3-yl]-6-methyl-phenoxy}-propane-1,2-diol; and
(S)-3-{2-Chloro-4-[5-(6-diethylamino-5-methyl-pyridin-3-yl)-[1,2,4]oxadiazol-3-yl]-6-methoxy-phenoxy}-propane-1,2-diol.

The compounds of Formula (I) according to embodiment i), or their pharmaceutically acceptable salts, can be used as medicaments, e.g. in the form of pharmaceutical compositions for enteral or parenteral administration, and are suitable for decreasing the number of circulating lymphocytes and for the prevention and/or treatment of diseases or disorders associated with an activated immune system.

The production of the pharmaceutical compositions can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the described compounds of Formula (I) or their pharmaceutically acceptable salts, optionally in combination with other therapeutically valuable substances, into a galenical administration form together with suitable, non-toxic, inert, pharmaceutically acceptable solid or liquid carrier materials and, if desired, usual pharmaceutical adjuvants.

The pharmaceutical compositions comprising a compound of Formula (I) are useful for the prevention and/or treatment of diseases or disorders associated with an activated immune system.

Such diseases or disorders include rejection of transplanted organs, tissue or cells; graft-versus-host diseases brought about by transplantation; autoimmune syndromes including rheumatoid arthritis; systemic lupus erythematosus; antiphospholipid syndrome; Hashimoto's thyroiditis; lymphocytic thyroiditis; multiple sclerosis; myasthenia gravis; type I diabetes; uveitis; episcleritis; scleritis; Kawasaki's disease, uveo-retinitis; posterior uveitis; uveitis associated with Behcet's disease; uveomeningitis syndrome; allergic encephalomyelitis; chronic allograft vasculopathy; post-infectious autoimmune diseases including rheumatic fever and post-infectious glomerulonephritis; inflammatory and hyperproliferative skin diseases; psoriasis; psoriatic arthritis; atopic dermatitis; myopathy; myositis; osteomyelitis; contact dermatitis; eczematous dermatitis; seborrhoeic dermatitis; lichen planus; pemphigus; bullous pemphigoid; epidermolysis bullosa; urticaria; angioedema; vasculitis; erythema; cutaneous eosinophilia; acne; scleroderma; alopecia areata; keratoconjunctivitis; vernal conjunctivitis; keratitis; herpetic keratitis; dystrophia epithelialis corneae; corneal leukoma; ocular pemphigus; Mooren's ulcer; ulcerative keratitis; scleritis; Graves' ophthalmopathy; Vogt-Koyanagi-Harada syndrome; sarcoidosis; pollen allergies; reversible obstructive airway disease; bronchial asthma; allergic asthma; intrinsic asthma; extrinsic asthma; dust asthma; chronic or inveterate asthma; late asthma and airway hyper-responsiveness; bronchiolitis; bronchitis; endometriosis; orchitis; gastric ulcers; ischemic bowel diseases; inflammatory bowel diseases; necrotizing enterocolitis; intestinal lesions associated with thermal burns; coeliac disease; proctitis; eosinophilic gastroenteritis; mastocytosis; Crohn's disease; ulcerative colitis; vascular damage caused by ischemic diseases and thrombosis; atherosclerosis; fatty heart; myocarditis; cardiac infarction; aortitis syndrome; cachexia due to viral disease; vascular thrombosis; migraine; rhinitis; eczema; interstitial nephritis; IgA-induced nephropathy; Goodpasture's syndrome; hemolytic-uremic syndrome; diabetic nephropathy; glomerulosclerosis; glomerulonephritis; tubulointerstitial nephritis; interstitial cystitis; multiple myositis; Guillain-Barré syndrome; Meniere's disease; polyneuritis; multiple neuritis; myelitis; mononeuritis; radiculopathy; hyperthyroidism; Basedow's disease; thyrotoxicosis; pure red cell aplasia; aplastic anemia; hypoplastic anemia; idiopathic thrombocytopenic purpura; autoimmune hemolytic anemia; autoimmune thrombocytopenia; agranulocytosis; pernicious anemia; megaloblastic anemia; anerythroplasia; osteoporosis; fibroid lung; idiopathic interstitial pneumonia; dermatomyositis; leukoderma vulgaris; ichthyosis vulgaris; photoallergic sensitivity; cutaneous T cell lymphoma; polyarteritis nodosa; Huntington's chorea; Sydenham's chorea; myocardosis; myocarditis; scleroderma; Wegener's granuloma; Sjogren's syndrome; adiposis; eosinophilic fascitis; lesions of gingiva, periodontium, alveolar bone, substantia ossea dentis; male pattern alopecia or alopecia senilis; muscular dystrophy; pyoderma; Sezary's syndrome; hypophysitis; chronic adrenal insufficiency; Addison's disease; ischemia-reperfusion injury of organs which occurs upon preservation; endotoxin shock; pseudomembranous colitis; colitis caused by drug or radiation; ischemic acute renal insufficiency; chronic renal insufficiency; lung cancer; malignancy of lymphoid origin; acute or chronic lymphocytic leukemias; lymphoma; pulmonary emphysema; cataracta; siderosis; retinitis pigmentosa; senile macular degeneration; vitreal scarring; corneal alkali burn; dermatitis erythema; ballous dermatitis; cement dermatitis; gingivitis; periodontitis; sepsis; pancreatitis; peripheral artery disease; carcinogenesis; solid cancer tumors; metastasis of carcinoma; hypobaropathy; autoimmune hepatitis; primary biliary cirrhosis; sclerosing cholangitis; partial liver resection; acute liver necrosis; cirrhosis; alcoholic cirrhosis; hepatic failure; fulminant hepatic failure; late-onset hepatic failure; and "acute-on-chronic" liver failure.

Preferred diseases or disorders to be treated and/or prevented with the compounds of Formula (I) according to embodiment i) are selected from the group consisting of rejection of transplanted organs such as kidney, liver, heart, lung, pancreas, cornea, and skin; graft-versus-host diseases brought about by stem cell transplantation; autoimmune syndromes including rheumatoid arthritis, multiple sclerosis, inflammatory bowel diseases such as Crohn's disease and ulcerative colitis, psoriasis, psoriatic arthritis, thyroiditis such as Hashimoto's thyroiditis, uveo-retinitis; atopic diseases such as rhinitis, conjunctivitis, dermatitis; asthma; type I diabetes; post-infectious autoimmune diseases including rheumatic fever and post-infectious glomerulonephritis; solid cancers and tumor metastasis.

Particularly preferred diseases or disorders to be treated and/or prevented with the compounds of Formula (I) according to embodiment i) are selected from the group consisting of rejection of transplanted organs selected from kidney, liver, heart and lung; graft-versus-host diseases brought about by stem cell transplantation; autoimmune syndromes selected from rheumatoid arthritis, multiple sclerosis, psoriasis, psoriatic arthritis, Crohn's disease, and Hashimoto's thyroiditis; and atopic dermatitis. Very preferably the diseases or disorders to be treated and/or prevented with the compounds of Formula (I) according to embodiment i) are selected from multiple sclerosis and psoriasis.

Furthermore, compounds of the Formula (I) according to embodiment i) are also useful, in combination with one or several immunomodulating agents, for the prevention and/or treatment of the diseases and disorders mentioned herein. According to a preferred embodiment of the invention, said agents are selected from the group consisting of immunosuppressants, corticosteroids, NSAID's, cytotoxic drugs, adhesion molecule inhibitors, cytokines, cytokine inhibitors, cytokine receptor antagonists and recombinant cytokine receptors.

The present invention also relates to the use of a compound of Formula (I) according to embodiment i) for the preparation of a pharmaceutical composition, optionally for use in combination with one or several immunomodulating agents, for the prevention or treatment of the diseases and disorders mentioned herein.

The compounds of Formula (I) can be manufactured by the methods given below, by the methods given in the Examples or by analogous methods. Optimum reaction conditions may vary with the particular reactants or solvents used, but such conditions can be determined by a person skilled in the art by routine optimisation procedures.

Compounds of the Formula (I) of the present invention can be prepared according to the general sequence of reactions outlined below. Only a few of the synthetic possibilities leading to compounds of Formula (I) are described.

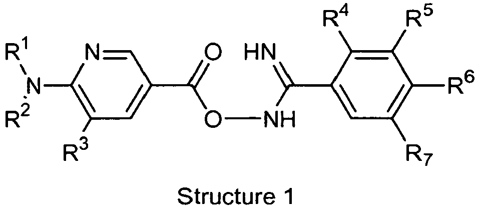

Compounds of Formula (I) which represent a 3-[1,2,4]oxadiazol-5-yl-pyridine derivative, are prepared by reacting a compound of Structure 1 in a solvent such as dioxane, THF, dimethoxyethane, xylene, toluene, benzene, pyridine, DMF, DCM, acetic acid, trifluoroacetic acid, etc. at rt or elevated temperatures in the presence or absence of auxiliaries such as acids (e.g. TFA, acetic acid, HCl, etc.), bases (e.g. NaH, NaOAc, Na₂CO₃, K₂CO₃, NEt₃, etc.), tetraalkylammonium salts, or water removing agents (e.g. oxalyl chloride, a carboxylic acid anhydride, POCl₃, PCl₅, P₄O₁₀, molecular sieves, methoxycarbonylsulfamoyl triethylammonium hydroxide (Burgess reagent), etc.) (Lit.: for conditions see experimental part or WO2007/080542 and references cited therein).

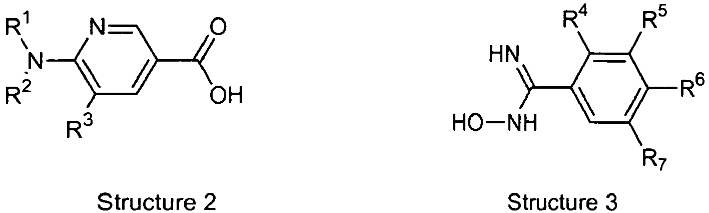

Compounds of Structure 1 may be prepared by reacting a compound of Structure 2 with a compound of Structure 3 in a solvent such as DMF, THF, DCM, etc. in the presence or absence of one or more coupling agents such as TBTU, DCC, EDC, HBTU, HOBt, CDI, PyBOP, etc. and in the presence or absence of a base such as NEt₃, DIPEA, NaH, K₂CO₃, etc. (see Lit. cited above).

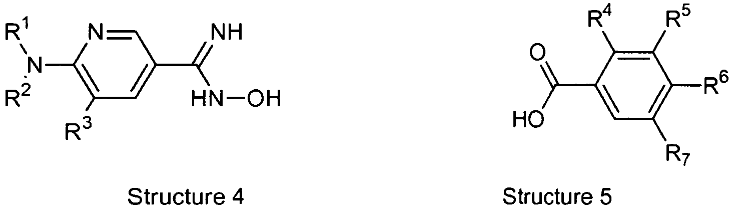

Compounds of Formula (I) which represent a 3-[1,2,4]oxadiazol-3-yl-pyridine derivative are prepared in an analogous fashion (see Lit. cited above) by reacting a compound of Structure 4 with a compound of Structure 5 and subsequent cyclisation of the corresponding hydroxyamidine ester intermediate.

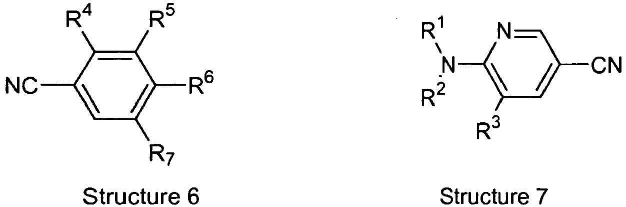

Compounds of Structure 3 and 4 may be prepared by reacting a compound of Structure 6 and 7, respectively, with hydroxylamine or one of its salts in a solvent such as methanol, ethanol, pyridine, etc. in the presence or absence of a base such as Na₂CO₃, K₂CO₃, NEt₃, KOtBu, etc. (see Lit. cited above).

Methods that effect the transformation of a compound of Structure 2 into a compound of Structure 7, or the opposite, are known to a person skilled in the art.

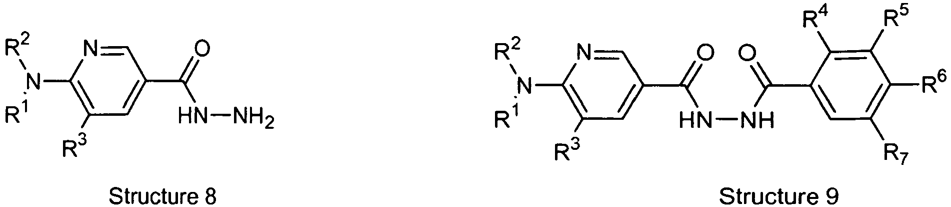

Compounds of Formula (I) which represent a 3-[1,3,4]oxadiazol-2-yl-pyridine derivative are prepared similarly by reacting a compound of Structure 2 with hydrazine (by using a coupling reagent such as TBTU, DCC, EDC, HBTU, PyPOB, HOBt, CDI, etc.) to form a compound of Structure 8 which is then coupled with a compound of Structure 5 to give a compound of Structure 9. A compound of Sturcture 9 can also be prepared by following the reverse reaction order i.e. by first coupling a compound of Stucture 5 with hydrazine followed by reacting the corresponding hydrazide intermediate with a compound of Structure 2. Dehydration of a compound of Structure 9 to form the desired 3-[1,3,4]oxadiazol-2-yl-pyridine derivative is affected by treating a compound of Structure 9 with a reagent such as POCl₃, CCl₄ or CBr₄ in combination with triphenylphosphine, P₂O₅, Burgess reagent, etc. in a solvent such as toluene, acetonitrile, dioxane, THF, CHCl₃, etc. at temperatures between 20 and 120°C in the presence or absence of microwave irradiation. (Lit. e.g. M. A. Garcia, S. Martin-Santamaria, M. Cacho, F. Moreno de la Llave, M. Julian, A. Martinez, B. De Pascual-Teresa, A. Ramos, J. Med. Chem. 48 (2005) 4068-4075, C. T. Brain, J. M. Paul, Y. Loong, P. J. Oakley, Tetrahedron Lett. 40 (1999) 3275-3278).

Depending on the nature of the functionalities present in the residues **R⁴** to **R⁷** in Structures 1, 3, 5, and 6, these functionalities may require temporary protection. Appropriate protecting groups are known to a person skilled in the art and include e.g. a benzyl or a trialkylsilyl group to protect an alcohol, a ketal to protect a diol, etc. These protecting groups may be employed according to standard methodology (e.g. T. W. Greene, P. G. M. Wuts, Protective Groups in Organic Synthesis, 3rd Edition, Wiley New York, 1991; P. J. Kocienski, Protecting Groups, Thieme Stuttgart, 1994). Alternatively, the desired residues **R⁴** to **R⁷**, in particular **R⁶**, may also be introduced in later steps that follow the coupling of the pyridine compounds of Structure 2, 4, or 8 with the phenyl derivatives of Stucture 3 or 5, respectively, by using a suitable precursor of a compound of Structure 3 or 5. The phenyl compounds of Structure 3, 5, and 6 or their precursors are either commercially available or are prepared according to procedures known to a person skilled in the art.

Alternatively, the bonds between the pyridine or the phenyl-ring and the central oxadiazole can also be formed by applying palladium catalysed cross coupling reactions. 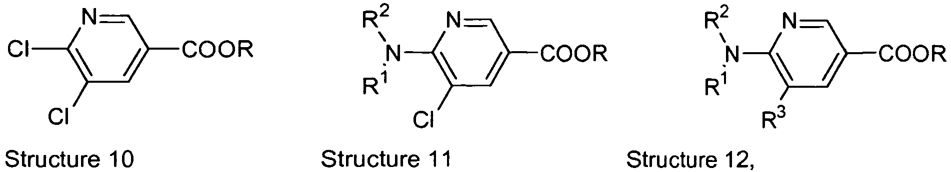 wherein R represents a C₁₋₄-alkyl group, preferably *iso*-propyl or *tert*.-butyl

Compounds of Structure 2 may be prepared preferrably by reacting a 5,6-dichloro-nicotinic acid ester (Structure 10) (prepared from commercially available 5,6-dichloro-nicotinic acid) with the appropriate amine NHR¹R² in the presence or absence of an additional solvent such as THF, dioxane, ethanol, etc, preferrably at temperatures above 50°C to give a compound of Structure 11. The compounds of Structure 11 can then be reacted with the appropriate alkyl-Zn reagent (e.g. Me₂Zn, MeZnCl, Et₂Zn, etc.) under Negishi reaction conditions (Lit.: e.g. H. Matsushita, E. Negishi J. Org. Chem. 47 (1982) 4161-4165) to give a compound of Structure 12, which can be hydrolysed to a compound of Structure 2. Alternatively, compounds of the Structure 12 may be prepared by reacting a compound of Structure 11 with an alkyl Grignard reagent in the presence of Fe(acac)₃ in a solvent such as THF, dioxane, DMF, NMP, etc., or combinations thereof, at temperatures ranging from -78 to 25°C (Fürstner conditions, Lit. e.g. A. Fürstner, A. Leitner, M. Mendez, H. Krause J. Am. Chem. Soc. 124 (2002) 13856-13863; A. Fürstner, A. Leitner Angew. Chem. 114 (2002) 632-635). In case R³ represents a C₂₋₃-alkyl group, the corresponding compounds of Structure 12 can also be prepared by reacting a compound of Structure 11 with an alkenyl boron derivative (e.g. 2,4,6-trivinyl-cyclotriboroxane) under Suzuki conditions (Lit.: e.g. F. Kerins, D. F. O'Shea J. Org. Chem. 67 (2002) 4968-4971). The obtained 6-amino-5-alkenyl-nicotinic acid derivative is hydrogenated to the corresponding compound of Structure 12.

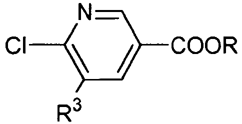

Structure 13, wherein R represents a C₁₋₄-alkyl group, preferably *iso*-propyl or *tert*.-butyl

Alternatively, the compounds of Structure 12 may also be prepared by reacting a compound of Structure 13 with the appropriate amine NHR¹R² in the presence or absence of a solvent such as methanol, ethanol, THF etc. at elevated temperatures or under Buchwald-Hartwig conditions (Lit.: J. P. Wolfe, H. Tomori, J. P. Sadighi, J. Yin, S. L. Buchwald J. Org. Chem. 65 (2000) 1158-1174; S. Wagaw, S. L. Buchwald J. Org. Chem. 61 (1996) 7240-7241; M. C. Harris, O. Geis, S. L. Buchwald J. Org. Chem. 64 (1999) 6019-6022; S. R. Stauffer, S. Lee, J. P. Stambuli, S. I. Hauck, J. F. Hartwig Org. Letters 2 (2000) 1423-1426).

The compound of Structure 13 wherein R³ represents a methyl group can be prepared from known 6-chloro-3-formyl-5-methyl-pyridine (Lit. e.g. EP-0702003) by oxidation of the formyl group to the carboxylic acid using oxidation reagents well known in the art such as aq. H₂O₂ in formic acid, KMnO₄, etc. in the presence or absence of a solvent such as toluene, THF, MeCN, acetone, etc. at temperatures between 0 and 120°C.

The residues R¹ and R² may also be introduced by sequencial alkylation and/or reductive amination of a compound of Structure 14 (Lit.: e.g. N. Finch, T. R. Campbell, C. W. Gemenden, H. J. Povalski J. Med. Chem. 23 (1980) 1405-1410; I.-C. Grig-Alexa; A.-L. Finaru; L. Ivan; P. Caubere; G. Guillaumet; Synthesis 2006, 619-628) which may be prepared by reacting a compound of Structure 13 or the corresponding acid with ammonia in a solvent such as water, methanol, ethanol, THF, etc. at elevated temperatures. 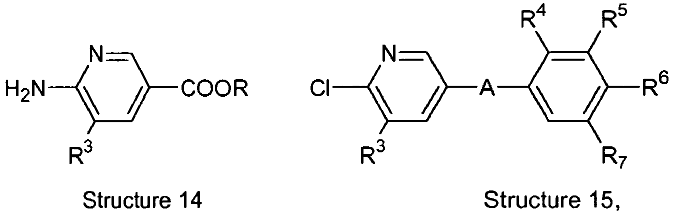 wherein R represents a C₁₋₄-alkyl group, preferably iso-propyl or *tert*.-butyl

In case R¹ represents hydrogen, the corresponding monoalkylamino-pyridine derivatives that may occur in the course of the synthesis of compounds of Formula (I), may require temporary protection at the secondary amine function.

The above described reaction sequences that allow the introduction of the two residues NR¹R² and R³ may also be applied to a compound in which the scaffold has already been further elaborated. For instance, the Buchwald reaction may also be applied to a compound of Structure 15.

Whenever the compounds of formula (I) are obtained in the form of mixtures of enantiomers, the enantiomers can be separated using methods known to one skilled in the art: e.g. by formation and separation of diastereomeric salts or by HPLC over a chiral stationary phase such as a Regis Whelk-O1(R,R) (10 µm) column, a Daicel ChiralCel OD-H (5-10 µm) column, or a Daicel ChiralPak IA (10 µm) or AD-H (5 µm) column. Typical conditions of chiral HPLC are an isocratic mixture of eluent A (EtOH, in presence or absence of an amine such as NEt₃, diethylamine) and eluent B (hexane), at a flow rate of 0.8 to 150 mL/min.

### Examples

The following examples illustrate the invention but do not at all limit the scope thereof.

All temperatures are stated in °C. Compounds are characterized by ¹H-NMR (300 MHz) or ¹³C-NMR (75 MHz) (Varian Oxford; chemical shifts are given in ppm relative to the solvent used; multiplicities: s = singlet, d = doublet, t = triplet, p = pentuplet, hex = hexet, hept = heptet, m = multiplet, br = broad, coupling constants are given in Hz); by LC-MS (Finnigan Navigator with HP 1100 Binary Pump and DAD, column: 4.6x50 mm, Zorbax SB-AQ, 5 µm, 120 A, gradient: 5-95% acetonitrile in water, 1 min, with 0.04% trifluoroacetic acid, flow: 4.5 mL/min), t_{R} is given in min; retention times or LC-MS marked with * refer to LC run under basic conditions, i.e. eluting with a gradient of MeCN in water containing 13 mM of ammonium hydroxide, otherwise identical conditions; retention times or LC-MS marked with ** refer to LC run under the following conditions: column: Ascentis express C18, 4.6 x 30 mm, gradient: 5-95% acetonitrile in water, 1 min, with 0.04% trifluoroacetic acid, flow: 4.5 mL/min; by TLC (TLC-plates from Merck, Silica gel 60 F₂₅₄); or by melting point. Compounds are purified by preparative HPLC (column: X-terra RP18, 50x19 mm, 5 µm, gradient: 10-95% acetonitrile in water containing 0.5 % of formic acid) or by MPLC (Labomatic MD-80-100 pump, Linear UVIS-201 detector, column: 350x18 mm, Labogel-RP-18-5s-100, gradient: 10% methanol in water to 100% methanol).

### Abbreviations (as used herein and in the description above):

- aq.: aqueous
- atm: atmosphere
- BSA: bovine serum albumin
- CC: column chromatography on silica gel
- CDI: carbonyl diimidazole
- DCC: dicyclohexyl carbodiimide
- DCM: dichloromethane
- DEAD: diethyl azodicarboxylate
- DIPEA: diisopropyl-ethylamine, Hünig's base, ethyl-diisopropylamine
- DMF: dimethylformamide
- DMSO: dimethylsulfoxide
- DPPP: 1,3-bis-(diphenylphosphino)-propane
- dppf: 1,1'-bis(diphenylphosphino)ferrocene
- EA: ethyl acetate
- EDC: N-(3-dimethylaminopropyl)-N'-ethyl-carbodiimide
- eq.: equivalent(s)
- Et: ethyl
- h: hour(s)
- HBTU: O-(benzotriazol-1-yl)-N,N,N',N'-tetramethyluronium hexafluorophosphate
- HOBt: 1-hydroxybenzotriazole
- HPLC: high performance liquid chromatography
- HV: high vacuum
- LC-MS: liquid chromatography - mass spectrometry
- Lit.: literature
- Me: methyl
- min: minute(s)
- MPLC: medium pressure liquid chromatography
- NaOAc: sodium acetate
- NEt₃: triethylamine
- NMP: N-methyl-pyrrolidone
- OAc: acetate
- Ph: phenyl
- prep.: preparative
- PyBOP: benzotriazol-1-yl-oxy-tris-pyrrolidino-phosphonium-hexafluoro-phosphate
- rt: room temperature
- sat.: saturated
- S1P: sphingosine 1-phosphate
- TBME: tertiary (tert.)-butyl methyl ether
- TBTU: 2-(1H-benzotriazole-1-yl)-1,2,3,3-tetramethyluronium tetrafluoroborate
- TFA: trifluoroacetic acid
- TH F: tetrahydrofuran
- TLC: thin layer chromatography
- t_{R}: retention time given in minutes

### 6-(Ethyl-methyl-amino)-5-methyl-nicotinic acid

a) To a solution of 5,6-dichloro nicotinic acid (12.2 g, 63.5 mmol) in isopropanol (70 mL), H₂SO₄ (4 mL) is added dropwise. The mixture is stirred at 80°C for 16 h before it is cooled to rt and concentrated in vacuo. The residue is dissolved in dioxane (100 mL) and concentrated again. The crude product is purified by CC (heptane:EA 1:3) to give 5,6-dichloro nicotinic acid isopropyl ester (9.29 g) as a pale beige oil; LC-MS: t_{R} = 1.33 min, [M+1]⁺ = 233.94.
b) A mixture of 5,6-dichloro nicotinic acid isopropyl ester (4.76 g, 22.3 mmol) and ethylmethylamine (6.88 g, 116.4 mmol) is stirred in a sealed vessel at 105°C for 72 h. The mixture is cooled to rt, diluted with EA (300 mL) and washed with sat. aq. NaHCO₃-solution (3x10 mL) followed by brine (10 mL). The organic extract is dried over MgSO₄, filtered, concentrated and dried to give 5-chloro-6-(ethyl-methyl-amino)-nicotinic acid isopropyl ester (5.18 g) as a yellow oil; LC-MS: t_{R} = 1.38 min, [M+1]⁺ = 257.02; ¹H NMR (D₆-DMSO): *δ* 1.19 (t, *J* = 6.8 Hz, 3 H), 1.30 (d, *J* = 6.0 Hz, 6 H), 3.08 (s, 3 H), 3.55 (q, *J* = 7.0 Hz, 2 H), 5.10 (hept, *J* = 6.3 Hz, 1 H), 7.98 (s, 1 H), 8.58 (s, 1 H).
c) A solution of 5-chloro-6-(ethyl-methyl-amino)-nicotinic acid isopropyl ester (5.18 g, 20.1 mmol), NMP (3.0 g, 30.2 mmol) and Fe(acac)₃ (498 mg, 1.41 mmol) in THF (150 mL) is put under argon before a methylmagnesium bromide (3.0 g, 25.2 mmol, solution in diethyl ether) is added dropwise. The dark red-brown solution turns yellow, then dark brown again. The mixture is stirred at rt for 2 h before another portion of methylmagnesium bromide (1.44 g, 12.1 mmol) is added. The dark mixture is stirred at rt for 16 h. Another portion of NMP (3.0 g, 30.2 mmol), Fe(acac)₃ (498 mg, 1.41 mmol) and methylmagnesium bromide (1.44 g, 12.1 mmol) is added and stirring is continued at rt for one more hour. The reaction mixture is diluted with EA (200 mL) and carefully quenched with ice-water (100 mL). The suspension is basified by adding 1 N aq. NaOH solution (10 mL) and filtered over a small pad of Hyflo and silica gel. The organic phase of the filtrate is separated and collected and the aq. is extracted with DCM (3x100 mL). The organic extracts are combined, dried over MgSO₄, filtered and concentrated. The crude product is purified on prep. MPLC on silica gel eluting with a gradient of EA in heptane to give 6-(ethyl-methyl-amino)-5-methyl-nicotinic acid isopropyl ester (2.19 g) as a beige oil; LC-MS: t_{R} = 0.76 min, [M+1]⁺ = 237.20.
d) A solution of 6-(ethyl-methyl-amino)-5-methyl-nicotinic acid isopropyl ester (2.19 g, 9.28 mmol) in THF (40 mL) and 25% aq. HCl (5 mL) is stirred at 65°C for 3 days before it is colled to rt and concentrated. The residue is dissolved in dioxane (50 mL) and concentrated again. This procedure is repeated one more time before the residue is dried under high vacuum to give the hydrochloride hydrate of the title compound (2.4 g) as a white powder; LC-MS: t_{R} = 0.68 min, [M+1]⁺ = 195.07; ¹H NMR (D₆-DMSO): *δ* 1.13 (t, *J* = 6.8 Hz, 3 H), 2.28 (s, 3 H), 2.93 (s, 3 H), 3.32 (q, *J* = 7.0 Hz, 2 H), 7.82 (s, 1 H), 8.52 (s, 1 H).

### 6-Diethylamino-5-methyl-nicotinic acid

a) Phosphoroxychloride (183 mL, 2 mol) is heated at 90°C and a mixture of commercially available 2-methyl-2-butennitrile (73 g, 0.9 mol) and DMF (154 mL, 2 mol) is added slowly while keeping the temperature at 100 to 110°C. The mixture is stirred at 110°C for 15 h, cooled to rt and diluted with DCM (500 mL). The mixture is cooled at 0°C and carefully quenched with water (500 mL). The phases are separated and the aq. phase extracted with DCM (total of 800 mL). The combined org. extracts are dried (Na₂SO₄), filtered and evaporated. The residue is crystallised from cyclohexane to provide 6-chloro-3-formyl-5-methyl-pyridine (28.3 g) as slightly yellow crystals; LC-MS: t_{R} = 0.76 min, [M+1]⁺ = 156.14.
b) A solution of 6-chloro-3-formyl-5-methyl-pyridine (10 g, 64 mmol) in formic acid (200 mL) is cooled at 0°C and an aq. 50% wt solution of H₂O₂ in water (9.6 mL, 360 mmol) is added at this temperature. The mixture is stirred at 0°C for 15 h, carefully diluted with water (200 mL) and extracted with DCM (8 x 100 mL). The combined org. extracts are washed with 1 M aq. HCl (100 mL) (check for remaining peroxide), dried (MgSO₄), filtered and evaporated. The residue is dried to give 6-chloro-5-methyl-nicotinic acid (9.56 g); LC-MS: t_{R} = 0.72 min, [M+1]⁺ = 172.0.
c) To a solution of 6-chloro-5-methyl-nicotinic acid (5.61 g, 32.7 mmol) in isopropanol (120 mL), trimethylchlorosilane (35.5 g, 327 mmol) is added dropwise. The mixture is stirred at 70°C for 16 h before it is cooled to rt, diluted with diethyl ether (500 mL) and washed with sat. aq. NaHCO₃-solution (5x50 mL). The washings are extracted back with diethyl ether (100 mL). The combined organic extracts are dried over MgSO₄, filtered and concentrated. The crude product is purified by CC (heptane:EA 9:1) to give 6-chloro-5-methyl-nicotinic acid isopropyl ester (4.32 g) as a yellow solid, LC-MS: t_{R} = 0.97 min, [M+1]⁺ = 214.10.
d) A mixture of 6-chloro-5-methyl-nicotinic acid isopropyl ester (1.40 g, 6.55 mmol) and diethylamine (4.80 g, 65.5 mmol) is stirred in a sealed vessel at 100°C for 2 weeks before it is cooled to rt, diluted with DCM (15 mL) and washed with 1 N aq. KHSO₄ solution (2x50 mL). The washings are extracted back with DCM (50 mL). The combined organic extracts are dried over Na₂SO₄, filtered and concentrated. The crude product is purified by CC (heptane:EA 9:1) to give 6-diethylamino-5-methyl-nicotinic acid isopropyl ester (111 mg) as pale yellow oil; LC-MS: t_{R} = 0.776 min, [M+1]⁺ = 251.19; ¹H NMR (CDCl₃): *δ* 1.17 (t, *J* = 7.0 Hz, 6 H), 1.36 (d, *J* = 6.3 Hz, 6 H), 2.31 (s, 3 H), 3.40 (q, *J* = 7.0 Hz, 4 H), 5.20-5.28 (m, 1 H), 7.89 (d, J = 2.3 Hz, 1 H), 8.74 (d, *J* = 2.3 Hz, 1 H).
e) A solution of the above ester (111 mg, 0.443 mmol) in 25% aq. HCl (10 mL) is stirred at 65°C for 18 h. The solvent is evaporated and the residue is dried under high vacuum to give the hydrochloride hydrate of the title compound (88 mg) as a white solid; LC-MS: t_{R} = 0.58 min, [M+1]⁺ = 209.10.

### 6-(Isopropyl-methyl-amino)-5-methyl-nicotinic acid

The title compound is prepared in analogy to 6-diethylamino-5-methyl-nicotinic acid using N-isopropyl-methyl-amine; LC-MS: t_{R} = 0.58 min, [M+1]⁺ = 209.10; ¹H NMR (D₆-DMSO): *δ* 1.23 (d, *J* = 6.5 Hz, 6 H), 2.40 (s, 3 H), 2.97 (s, 3 H), 4.22 (hept, *J* = 6.8 Hz, 1 H), 8.07 (s, 1 H), 8.43 (d, *J* = 2.0 Hz, 1 H).

### 5-Methyl-6-pyrrolidin-1-yl-nicotinic acid

The title compound is prepared in analogy to 6-diethylamino-5-methyl-nicotinic acid using pyrrolidine; LC-MS: t_{R} = 0.55 min, [M+1]⁺ = 207.10; ¹H NMR (D₆-DMSO): *δ* 1.94-2.00 (m, 4 H), 2.54 (s, 3 H), 3.79-3.85 (m, 4 H), 7.99 (s, 1 H), 8.28 (s, 1 H).

### 6-Diethylamino-5-ethyl-nicotinic acid

a) To a solution of 5,6-dichloronicotinic acid (10.0 g, 50.0 mmol) in THF (600 mL), triphenylphosphine (19.67 g, 75.0 mmol) and ethanol (5.55 g, 75.0 mmol) is added. The mixture is cooled to 0°C before DEAD (32.65 g, 75.0 mmol) is added. The mixture is stirred and warmed to rt. Stirring is continued for 16 h before sat. aq. NaHCO₃ solution is added. The mixture is repeatedly extracted with EA. The combined organic extracts are dried over MgSO₄, filtered and concentrated. The crude product is purified by CC (heptane:EA 7:3) to give 5,6-dichloronicotinic acid ethyl ester (11.4 g) as a white solid; LC-MS: t_{R} = 0.96 min, [M+1]⁺ = 220.02.
b) A mixture of 5,6-dichloronicotinic acid ethyl ester (2.91 g, 15.2 mmol) and diethyl-amine (11.1 g, 152 mmol) is stirred in a sealed vessel at 80°C for 72 h. The mixture is cooled to rt and concentrated. The residue is dissolved in DCM (15 mL) and washed with 1 N aq. KHSO₄ solution (2x50 mL). The washings are extracted back with DCM (50 mL). The combined organic extracts are dried over Na₂SO₄, filtered, concentrated and dried to give 5-chloro-6-diethylamino-nicotinic acid ethyl ester (3.36 g) as a yellow oil; LC-MS: t_{R} = 1.08 min, [M+1]⁺ = 257.12; ¹H NMR (CDCl₃): *δ* 1.26 (t, *J* = 7.0 Hz, 6 H), 1.39 (t, *J* = 7.3 Hz, 3 H), 3.62 (q, J = 7.0 Hz, 4 H), 4.36 (q, *J* = 7.3 Hz, 2 H), 8.07 (s, 1 H), 8.70 (s, 1 H).
c) To a solution of 5-chloro-6-diethylamino-nicotinic acid ethyl ester (2.96 g, 11.5 mmol) in dioxane (50 mL), Pd(dppf) (470 mg, 0.576 mmol) is added under argon. To this mixture, diethyl zinc (8.53 g, 69.1 mmol, as a 1.1 M solution in toluene) is added dropwise. The mixture is stirred at 75°C for 16 h before another portion of Pd(dppf) 94 mg, 0.115 mmol) and diehtyl zinc (1.42 g, 11.5 mmol, as a 1.1 M solution in toluene) is added. Stirring is continued at 75°C for 24 h. The reaction mixture is cooled to rt and carefully quenched with water. The mixture is filtered over celite and the filtrate is extracted twice with EA. The combined organic extracts are dried over MgSO₄, filtered and concentrated. The crude product is purified by CC (heptane:EA 9:1) to give 6-diethylamino-5-ethyl-nicotinic acid ethyl ester (2.40 g) as a colourless oil; LC-MS: t_{R} = 0.78 min, [M+1]⁺ = 251.19; ¹H NMR (CDCl₃): *δ* 1.15 (t, *J* = 7.0 Hz, 6 H), 1.27 (t, *J* = 7.3 Hz, 3 H), 1.40 (t, *J* = 7.3 Hz, 3 H), 2.65 (q, *J* = 7.5 Hz, 2 H), 3.36 (q, *J* = 7.0 Hz, 4 H), 4.37 (q, *J* = 7.0 Hz, 2 H), 7.99 (d, *J* = 2.3 Hz, 1 H), 8.76 (d, *J* = 2.3 Hz, 1 H).
d) A solution of 6-diethylamino-5-ethyl-nicotinic acid ethyl ester (1.78 g, 5.34 mmol) in 25% aq. HCl (50 mL) is stirred at 65°C for 18 h. The solvent is evaporated and the product is dried under high vacuum to give the hydrochloride hydrate of the title compound (2.30 g) as a white solid; LC-MS: t_{R} = 0.62 min, [M+1]⁺ = 223.15.

### 5-Ethyl-6-(isopropyl-methyl-amino)-nicotinic acid

The title compound is prepared in analogy to 6-diethylamino-5-ethyl-nicotinic acid using ispropyl-methylamine; LC-MS: t_{R} = 0.64 min, [M+1]⁺ = 223.14.

### 5-Ethyl-6-pyrrolidin-1-yl-nicotinic acid

The title compound is prepared in analogy to diethylamino-5-ethyl-nicotinic acid using pyrrolidine; LC-MS: t_{R} = 0.59 min, [M+1]⁺ = 221.13; ¹H NMR (CDCl₃): *δ* 1.23 (t, *J* = 7.5 Hz, 3 H), 1.96-2.04 (m, 4 H), 2.78 (q, *J* = 7.5 Hz, 2 H), 3.68-3.76 (m, 4 H), 7.93 (s, 1 H), 8.77 (d, *J* = 1.5 Hz, 1 H).

### 3-Ethyl-4-hydroxy-5-methyl-benzonitrile

The title compound is prepared from 3-ethyl-4-hydroxy-5-methyl-benzaldehyde following literature procedures (A. K. Chakraborti, G. Kaur, Tetrahedron 55 (1999) 13265-13268); LC-MS: t_{R} = 0.90 min; ¹H NMR (CDCl₃): δ 1.24 (t, *J* = 7.6 Hz, 3 H), 2.26 (s, 3 H), 2.63 (q, *J* = 7.6 Hz, 2 H), 5.19 (s, 1 H), 7.30 (s, 2 H).

### 3-Chloro-4-hydroxy-5-methyl-benzonitrile

The title compound is prepared from commercially available 2-chloro-6-methyl-phenol in analogy to literature procedures (see 3-ethyl-4-hydroxy-5-methyl-benzonitrile); LC-MS: t_{R} = 0.85 min. ¹H NMR (CDCl₃): δ 2.33 (s, 3 H), 6.10 (s, 1 H), 7.38 (s, 1 H), 7.53 (d, *J* = 1.8 Hz, 1 H).

### 4-Hydroxy-2-methoxy-benzonitrile

The title compound is prepared from commercially available 4-hydroxy-2-methoxybenzaldehyde in analogy to literature procedures (see 3-ethyl-4-hydroxy-5-methyl-benzonitrile); LC-MS: t_{R} = 0.74 min. ¹H NMR (D₆-DMSO): δ 3.84 (s, 3 H), 6.47 (d, *J* = 8.5 Hz, 1 H), 6.54 (s, 1 H), 7.49 (d, *J* = 8.5 Hz, 1 H), 10.6 (s, 1 H).

### 3-Chloro-4-hydroxy-5-methoxy-benzonitrile

The title compound is prepared from commercially available 3-chloro-4-hydroxy-5-methoxybenzaldehyde in analogy to literature procedures (see 3-ethyl-4-hydroxy-5-methyl-benzonitrile); LC-MS: t_{R} = 0.82 min; ¹H NMR (CDCl₃): *δ* 3.98 (s, 3 H), 6.36 (s, 1 H), 7.04 (s, 1 H), 7.34 (s, 1 H).

### 4,N-Dihydroxy-3,5-dimethyl-benzamidine

The title compound is prepared from commercially available 4-hydroxy-3,5-dimethyl-benzonitrile according to literature procedures (e.g. E. Meyer, A. C. Joussef, H. Gallardo, Synthesis 2003, 899-905); ¹H NMR (CD₃OD): δ 7.20 (s, 2H), 2.20 (s, 6H).

### 3-Ethyl-4,N-dihydroxy-5-methyl-benzamidine

The title compound is prepared from commercially available 2-ethyl-6-methyl-phenol following literature procedures (G. Trapani, A. Latrofa, M. Franco, C. Altomare, E. Sanna, M. Usala, G. Biggio, G. Liso, J. Med. Chem. 41 (1998) 1846-1854; A. K. Chakraborti, G. Kaur, Tetrahedron 55 (1999) 13265-13268; E. Meyer, A. C. Joussef, H. Gallardo, Synthesis 2003, 899-905); LC-MS: t_{R} = 0.55 min; ¹H NMR (D₆-DMSO): δ 9.25 (s br, 1H), 7.21 (s, 2H), 5.56 (s, 2H), 2.55 (q, J = 7.6 Hz, 2H), 2.15 (s, 3H), 1.10 (t, J = 7.6 Hz, 3H).

### 4,N-Dihydroxy-3-methyl-5-propyl-benzamidine

The title compound is prepared from commercially available 2-methyl-6-propyl-phenol in analogy to literature procedures (e.g B. Roth et al. J. Med. Chem. 31 (1988) 122-129; and literature cited for 3-ethyl-4,N-dihydroxy-5-methyl-benzamidine); LC-MS: t_{R} = 0.54 min; [M+1]⁺ = 209.43; ¹H NMR (D₆-DMSO): *δ* 0.90 (t, *J* = 7.3 Hz, 3 H), 1.48-1.59 (m, 3 H), 2.19 (s, 3 H), 2.56 (t, *J* = 7.3 Hz, 2H), 7.37 (s, 1 H), 7.40 (s, 1 H), 9.34 (s, 1 H).

### 3-Chloro-4,N-dihydroxy-5-methyl-benzamidine

The title compound is prepared from commercially available 2-chloro-6-methyl-phenol in analogy to literature procedures (e.g B. Roth et al. J. Med. Chem. 31 (1988) 122-129; and literature cited for 3-ethyl-4,N-dihydroxy-5-methyl-benzamidine); 3-chloro-4-hydroxy-5-methyl-benzaldehyde: LC-MS: t_{R} = 0.49 min; [M+1]⁺ = 201.00; ¹H NMR *δ* 2.24 (s, 2 H), 2.35 (s, 4 H), 5.98 (s br, 1 H), 7.59 (d, *J* = 1.8 Hz, 1 H), 7.73 (d, *J* = 1.8 Hz, 1 H), 9.80 (s, 1 H); 3-chloro-4,N-dihydroxy-5-methyl-benzamidine: ¹H NMR (D₆-DMSO): *δ* 2.21 (s, 3 H), 5.72 (s br, 2 H), 7.40 (s, 1 H), 7.48 (s, 1 H), 9.29 (s br, 1 H), 9.48 (s br, 1 H).

### 3-[4-(N-Hydroxycarbamimidoyl)-2,6-dimethyl-phenyl]-propionic acid tert-butyl ester

a) To an ice-cooled solution of 4-hydroxy-3,5-dimethyl-benzoic acid methyl ester (7.52 g, 41.7 mmol) in DCM (250 mL) and pyridine (10 mL), trifluoromethanesulfonic acid anhydride (13.0 g, 45.9 mmol) is added over a period of 20 min. Upon complete addition, the ice bath is removed and the reaction is stirred for further 1 h at rt. The mixture is diluted with DCM (150 mL), washed with 10% aq. citric acid solution followed by brine, dried over MgSO₄, filtered and evaporated. The residue is purified by CC (heptane:EA 9:1) to give 3,5-dimethyl-4-trifluoromethanesulfonyloxy-benzoic acid methyl ester (11.8 g) as colourless fine needles; LC-MS: t_{R} = 1.08 min.
b) To a stirred solution of the above triflate (11.8 g, 37.8 mmol) in dry DMF (155 mL) is sequentially added NEt₃ (7.6 g, 75.6 mmol), tert.-butyl acrylate (48.4 g, 378 mmol), DPPP (779 mg, 1.89 mmol) and Pd(OAc)₂ (424 mg, 1.89 mmol) under nitrogen. The mixture is stirred at 115°C for 18 h before another portion of DPPP (160 mg, 0.39 mmol) and Pd(OAc)₂ (80 mg, 0.36 mmol) is added. Stirring is continued for 4 h at 115°C before the mixture is cooled to rt, diluted with diethyl ether (350 mL) and washed with 1 N aq. HCl, followed by sat. aq. NaHCO₃ solution. The org. extract is dried over MgSO₄, filtered and evaporated. The residue is purified by CC (heptane:EA 4:1) to give 4-(2-tert-butoxycarbonyl-vinyl)-3,5-dimethyl-benzoic acid methyl ester (11.21 g) as a colourless solid; LC-MS: t_{R} = 1.09 min.
c) To a solution of 4-(2-tert-butoxycarbonyl-vinyl)-3,5-dimethyl-benzoic acid methyl ester (11.2 g, 38.6 mmol) in ethanol (50 mL) and THF (50 mL), Pd/C (1.0 g, 10% Pd) is added. The mixture is stirred for 16 h at rt under 2.5 bar of H₂. The catalyst is filtered off and the filtrate is concentrated and dried under HV to give 4-(2-tert-butoxycarbonyl-ethyl)-3,5-dimethyl-benzoic acid methyl ester (10.8 g) as a colourless oil; LC-MS: t_{R} = 1.08 min.
d) To a solution of 4-(2-tert-butoxycarbonyl-ethyl)-3,5-dimethyl-benzoic acid methyl ester (10.8 g, 37.0 mmol) in ethanol (100 mL) a 2 M aq. solution of LiOH (50 mL) is added at 0°C. The turbid mixture is stirred at 0°C for 30 min, then at rt for 4 h. The mixture is diluted with 10% aq. citric acid solution and extracted three times with diethyl ether. The combined org. extracts are dried over MgSO₄, filtered and concentrated. The solid residue is suspended in diethyl ether/heptane, stirred at rt, and filtered. The slurry procedure in diethyl ether/heptane is repeated. The solid material is collected and dried under HV to give 4-(2-tert-butoxycarbonyl-ethyl)-3,5-dimethyl-benzoic acid (5.09 g) as a white crystalline powder; LC-MS: t_{R} = 0.95 min, [M+1]⁺ = 279.14; ¹H NMR (CDCl₃): δ 1.47 (s, 9 H), 2.30-2.40 (m, 2 H), 2.39 (s, 6 H), 2.94-3.03 (m, 2 H), 7.75 (s, 2 H).
e) To a suspension of 4-(2-tert-butoxycarbonyl-ethyl)-3,5-dimethyl-benzoic acid (8.00 g, 28.7 mmol) in isopropanol (100 mL), HOBt (4.27 g, 31.6 mmol) followed by EDC hydrochloride (6.34 g, 33.1 mmol) is added. After stirring at rt for 1 h, 25% aq. ammonia (16.1 mL) is added. Stirring is continued for 30 min before the isopropanol is evaporated under reduced pressure. The remaining solution is diluted with isopropyl acetate (200 mL), washed three times with approximately 0.5 N aq. NaHCO₃ solution (100 mL) followed by water (50 mL), dried over MgSO₄, filtered, concentrated and dried to give 3-(4-carbamoyl-2,6-dimethylphenyl)-propionic acid tert-butyl ester (7.5 g) as an off-white solid.
f) To an ice-cooled solution of 3-(4-carbamoyl-2,6-dimethyl-phenyl)-propionic acid tert-butyl ester (7.00 g, 25.2 mmol) and NEt₃ (7.66 g, 75.7 mmol) in DCM (100 mL), trifluoroacetic anhydride (6.06 g, 28.8 mmol) is added slowly so that the reaction temperature stays below 15°C. The clear yellow solution is stirred at rt for 1 h before it is washed twice with water (100 mL) and concentrated. The crude product is purified by recrystallisation from methanol to give 3-(4-cyano-2,6-dimethyl-phenyl)-propionic acid tert-butyl ester (4.2 g) as a white solid, ¹H NMR (CDCl₃): *δ* 1.48 (s, 9 H), 2.33-2.37 (m, 2 H), 2.38 (s, 6 H), 2.94-3.01 (m, 2 H), 7.31 (s, 2 H).
g) A solution of 3-(4-cyano-2,6-dimethyl-phenyl)-propionic acid tert-butyl ester (4.1 g, 15.8 mmol), hydroxylamine hydrochloride (1.65 g, 23.7 mmol) and NEt₃ (3.20 g, 31.6 mmol) in methanol (40 mL) is refluxed for 2 h before the solvent is removed in vacuo. The residue is taken up in isopropyl acetate (50 mL) and washed twice with water (50 mL). The org. extract is dried over MgSO₄, filtered, evaporated and dried to give 3-[4-(N-hydroxycarbamimidoyl)-2,6-dimethyl-phenyl]-propionic acid tert-butyl ester (4.4 g) as a white solid.

### 3-[2-Ethyl-4-(N-hydroxycarbamimidoyl)-6-methyl-phenyl]-propionic acid tert-butyl ester

The title compound is prepared in analogy to 3-[4-(N-hydroxycarbamimidoyl)-2,6-dimethylphenyl]-propionic acid tert-butyl ester; ¹H NMR (CDCl₃): *δ* 1.26 (t, *J* = 7.5 Hz, 3 H), 2.34-2.41 (m, 5 H), 2.70 (q, *J* = 7.8 Hz, 2 H), 2.94-3.01 (m, 2 H), 4.85 (s br, 1 H), 7.28 (s, 1 H), 7.32 (s, 1 H).

### rac-4-(2,2-Dimethyl-[1,3]dioxolan-4-ylmethoxy)-N-hydroxy-3,5-dimethyl-benzamidine

a) To a solution of 3,5-dimethyl-4-hydroxy-benzonitrile (5.0 g, 34.0 mmol) in THF (40 mL), rac-(2,2-dimethyl-[1,3]dioxolan-4-yl)-methanol (4.49 g, 34.0 mmol) followed by triphenylphosphine (13.4 g, 50.9 mmol) is added. The mixture is cooled with an ice-bath before DEAD (8.87 g, 50.9 mmol, 23.4 mL of a 40% solution in toluene) is added dropwise. The mixture is stirred at rt for 1 h, the solvent is removed in vacuo and the residue is purified by CC (heptane:EA 99:1 to 92:8) to give rac-4-(2,2-dimethyl-[1,3]dioxolan-4-ylmethoxy)-3,5-dimethyl-benzonitrile (7.20 g) as a pale yellow oil; LC-MS: t_{R} = 0.99 min, [M+1]⁺ = not detected.
b) To a solution of potassium tert.-butylate (6.18 g, 55.1 mmol) in methanol (125 mL), hydroxylamine hydrochloride (5.74 g, 82.7 mmol) is added. To this solution, a solution of rac-4-(2,2-dimethyl-[1,3]dioxolan-4-ylmethoxy)-3,5-dimethyl-benzonitrile (7.20 g, 27.6 mmol) in methanol (40 mL) is added. The mixture is refluxed for 72 h before the solvent is removed in vacuo. The residue is purified by prep. HPLC (XBridge Prep C18, 30x75 mm, 5 µm, 2-95% acetonitrile in water containing 0.5% sat. aq. NH₃) to give the title compound (4.85 g) as a pale yellow solid; LC-MS: t_{R} = 0.67 min, [M+1]⁺ = 295.06; ¹H NMR (CDCl₃): *δ* 1.43 (s, 3 H), 1.48 (s, 3 H), 2.29 (s, 6 H), 3.76-3.81 (m, 1 H), 3.83-3.88 (m, 1 H), 3.93-3.99 (m, 1 H), 4.17-4.23 (m, 1 H), 4.47-4.54 (m, 1 H), 5.02 (s br, 1 H), 7.28 (s, 2H).

### (S)-4-(2,2-Dimethyl-[1,3]dioxolan-4-ylmethoxy)-N-hydroxy-3,5-dimethyl-benzamidine

The title compound is prepared in analogy to rac-4-(2,2-dimethyl-[1,3]dioxolan-4-ylmethoxy)-N-hydroxy-3,5-dimethyl-benzamidine using (S)-(2,2-dimethyl-[1,3]dioxolan-4-yl)-methanol; LC-MS: t_{R} = 0.67 min, [M+1]⁺ = 295.01.

### (R)-3-Chloro-4-(2,2-dimethyl-[1,3]dioxolan-4-ylmethoxy)-N-hydroxy-5-methyl-benzamidine

The title compound is obtained as a colorless oil (1.39 g) in analogy to rac-4-(2,2-dimethyl-[1,3]dioxolan-4-ylmethoxy)-N-hydroxy-3,5-dimethyl-benzamidine starting from 3-chloro-4-hydroxy-5-methyl-benzonitrile and L-alpha-beta-isopropyliden glycerol; LC-MS: t_{R} = 0.66 min, [M+H]⁺ = 314.96.

### (R)-4-(2,2-Dimethyl-[1,3]dioxolan-4-ylmethoxy)-N-hydroxy-2-methoxy-benzamidine

The title compound is obtained as a beige oil (2.46 g) in analogy to rac-4-(2,2-dimethyl-[1,3]dioxolan-4-ylmethoxy)-N-hydroxy-3,5-dimethyl-benzamidine starting from 4-hydroxy-2-methoxy-benzonitrile and L-alpha-beta-isopropyliden glycerol; LC-MS: t_{R} = 0.62 min, [M+H]⁺ = 296.97.

### (R)-3-Chloro-4-(2,2-dimethyl-[1,3]dioxolan-4-ylmethoxy)-N-hydroxy-5-methoxy-benzamidine

The title compound is prepared in analogy to rac-4-(2,2-dimethyl-[1,3]dioxolan-4-ylmethoxy)-N-hydroxy-3,5-dimethyl-benzamidine starting from 3-chloro-4-hydroxy-5-methoxy-benzonitrile and L-α,β-isopropyliden glycerol; LC-MS: t_{R} = 0.42 min, [M+H]⁺ = 331.17; ¹H NMR (D₆-DMSO): *δ* 1.30 (s, 3 H), 1.34 (s, 3 H), 3.86 (s, 3 H), 3.87-3.93 (m, 2 H), 4.00-4.12 (m, 2 H), 4.36 (quint, *J* = 5.8 Hz, 1 H), 5.90 (s, 2 H), 7.32 (d, *J* = 2.0 Hz, 1 H), 7.34 (d, *J* = 2.0 Hz, 1 H), 9.71 (s, 1 H).

### rac-2-Hydroxy-N-{2-hydroxy-3-[4-(N-hydroxycarbamimidoyl)-2,6-dimethyl-phenoxy]-propyl}-acetamide

The title compound is prepared in analogy to N-((S)-3-[2-ethyl-4-(N-hydroxycarbamimidoyl)-6-methyl-phenoxy]-2-hydroxy-propyl)-2-hydroxy-acetamide; LC-MS: t_{R} = 0.48 min, [M+1]⁺ = 312.05; ¹H NMR (D₆-DMSO): *δ* 2.21 (s, 6 H), 3.14-3.25 (m, 1 H), 3.35-3.46 (m, 1 H), 3.60-3.69 (m, 2 H), 3.80 (s, 2 H), 3.85-3.94 (m, 1 H), 5.69 (s br, 2 H), 7.30 (s, 2 H), 7.63 (t, *J* = 5.6 Hz, 1 H), 8.11 (s, 1 H).

### (S)-4-(3-Amino-2-hydroxypropoxy)-3-ethyl-5-methylbenzonitrile

a) To a solution of 3-ethyl-4-hydroxy-5-methyl-benzonitrile (5.06 g, 31.4 mmol) in THF (80 mL), PPh₃ (9.06 g, 34.5 mmol) and (R)-glycidol (2.29 mL, 34.5 mmol) are added. The mixture is cooled to 0°C before DEAD in toluene (15.8 mL, 34.5 mmol) is added. The mixture is stirred for 18 h while warming up to rt. The solvent is evaporated and the crude product is purified by CC (heptane:EA 7:3) to give 3-ethyl-5-methyl-4-oxiranylmethoxy-benzonitrile (5.85 g) as a yellow oil; LC-MS: t_{R} = 0.96 min; [M+42]⁺ = 259.08.
b) The above epoxide is dissolved in 7 N NH₃ in methanol (250 mL) and the solution is stirred at 65°C for 18 h. The solvent is evaporated to give crude (S)-4-(3-amino-2-hydroxypropoxy)-3-ethyl-5-methylbenzonitrile (6.23 g) as a yellow oil; LC-MS: t_{R} = 0.66 min; [M+1]⁺ = 235.11.

### N-((S)-3-[2-Ethyl-4-(N-hydroxycarbamimidoyl)-6-methyl-phenoxy]-2-hydroxy-propyl)-2-hydroxy-acetamide

a) To a solution of (S)-4-(3-amino-2-hydroxypropoxy)-3-ethyl-5-methylbenzonitrile (6.23 g, 26.59 mmol), glycolic acid (2.43 g, 31.9 mmol), HOBt (4.31 g, 31.9 mmol), and EDC hydrochloride (6.12 g, 31.9 mmol) are added. The mixture is stirred at rt for 18 h before it is diluted with sat. aq. NaHCO₃ and extracted twice with EA. The combined organic extracts are dried over MgSO₄, filtered and concentrated. The crude product is purified by CC with DCM containing 8% of methanol to give (S)-N-[3-(4-cyano-2-ethyl-6-methyl-phenoxy)-2-hydroxypropyl]-2-hydroxy-acetamide (7.03 g) as a yellow oil; LC-MS: t_{R} = 0.74 min; [M+1]⁺ = 293.10; ¹H NMR (CDCl₃): δ 1.25 (t, *J* = 7.5 Hz, 3 H), 2.32 (s, 3 H), 2.69 (q, *J* = 7.5 Hz, 2 H), 3.48-3.56 (m, 3 H), 3.70-3.90 (m, 3 H), 4.19 (s, br, 3 H), 7.06 (m, 1 H), 7.36 (s, 1 H), 7.38 (s, 1 H).
b) The above nitrile is converted to the N-hydroxy-benzamidine according to literature procedures (e.g. E. Meyer, A. C. Joussef, H. Gallardo, *Synthesis* **2003,** 899-905); LC-MS: t_{R} = 0.51 min; [M+1]⁺ = 326.13; ¹H NMR (D₆-DMSO): δ 1.17 (t, *J* 7.4Hz, 3 H), 2.24 (s, 3H), 2.62 (q, *J* 7.4Hz, 2 H), 3.23 (m, 1 H), 3.43 (m, 1 H), 3.67 (m, 2 H), 3.83 (s, 2 H), 3.93 (m, 1 H), 5.27 (s br, 1 H), 5.58 (s br, 1 H), 5.70 (s, 2 H), 7.34 (s, 1 H), 7.36 (s, 1 H), 7.67 (m, 1 H), 9.46 (s br, 1H).

### (S)-2-Hydroxy-N-{2-hydroxy-3-[4-(N-hydroxycarbamimidoyl)-2,6-dimethyl-phenoxy]-propyl}-acetamide

The title compound is prepared in analogy to N-((S)-3-[2-ethyl-4-(N-hydroxycarbamimidoyl)-6-methyl-phenoxy]-2-hydroxy-propyl)-2-hydroxy-acetamide; LC-MS: t_{R} = 0.23 min, [M+1]⁺ = 312.25.

### (S)-N-(3-[2-Chloro-4-(N-hydroxycarbamimidoyl)-6-methyl-phenoxy]-2-hydroxy-propyl)-2-hydroxy-acetamide

The title compound is obtained as a beige wax (1.1 g) in analogy to N-((S)-3-[2-ethyl-4-(N-hydroxycarbamimidoyl)-6-methyl-phenoxy]-2-hydroxy-propyl)-2-hydroxy-acetamide starting from 3-chloro-4-hydroxy-5-methyl-benzonitrile; LC-MS: t_{R} = 0.48 min, [M+H]⁺ = 331.94.

### (S)-2-Hydroxy-N-(2-hydroxy-3-[4-(N-hydroxycarbamimidoyl)-3-methyl-phenoxy]-propyl)-acetamide

The title compound is obtained as a beige oil (1.0 g) in analogy to N-((S)-3-[2-ethyl-4-(N-hydroxycarbamimidoyl)-6-methyl-phenoxy]-2-hydroxy-propyl)-2-hydroxy-acetamide starting from 4-hydroxy-2-methyl-benzonitrile; LC-MS: t_{R} = 0.35 min, [M+H]⁺ = 297.99.

### (S)-2-Hydroxy-N-(2-hydroxy-3-[4-(N-hydroxycarbamimidoyl)-2-methoxy-6-methyl-phenoxy]-propyl)-acetamide

The title compound is obtained as a reddish oil (1.3 g) in analogy to N-((S)-3-[2-ethyl-4-(N-hydroxycarbamimidoyl)-6-methyl-phenoxy]-2-hydroxy-propyl)-2-hydroxy-acetamide starting from 4-hydroxy-3-methoxy-5-methyl-benzonitrile; LC-MS: t_{R} = 0.49 min, [M+H]⁺ = 327.98.

### 4-Benzyloxy-3-ethyl-5-methyl-benzoic acid

a) To an ice-cold solution of H₂SO₄ (150 mL) in water (250 mL) 2-ethyl-6-methylaniline (15.0 g, 111 mmol) is added. The solution is treated with ice (150 g) before a solution of NaNO₂ (10.7 g, 155 mmol) in water (150 mL) and ice (50 g) is added dropwise. The mixture is stirred at 0°C for 1 h. 50% aq. H₂SO₄ (200 mL) is added and stirring is continued at rt for 18 h. The mixture is extracted with DCM, the org. extracts are dried over MgSO₄ and evaporated. The crude product is purified by CC eluting with heptane:EA 9:1 to give 2-ethyl-6-methyl-phenol (8.6 g) as a crimson oil; LC-MS: t_{R} = 0.89 min; ¹H NMR (CDCl₃): δ 7.03-6.95 (m, 2H), 6.80 (t, J = 7.6 Hz, 1H), 4.60 (s, 1H), 2.64 (q, J = 7.6 Hz, 2H), 2.25 (s, 3H), 1.24 (t, J = 7.6 Hz, 3H).
b) A solution of 2-ethyl-6-methyl-phenol (8.40 g, 61.7 mmol) and hexamethylene tetraamine (12.97 g, 92.5 mmol) in acetic acid (60 mL) and water (14 mL) is heated to 115°C. The water is distilled off at 117°C and collected with a Dean-Stark apparatus. Then the water separator is replaced by a reflux condensor and the mixture is refluxed for 3 h. The mixture is cooled to rt, diluted with water (100 mL) and extracted with EA. The org. extract is washed with sat. aq. NaHCO₃, dried over MgSO₄ and evaporated. The remaining solid is dissolved in EA and treated with heptane to initialize crystallisation. The solid material is collected and dried to give 3-ethyl-4-hydroxy-5-methyl-benzaldehyde (3.13 g) as a colourless crystalline powder, ¹H NMR (CDCl₃): δ 9.83 (s, 1H), 7.58-7.53 (m, 2H), 5.30 (s br, 1H), 2.69 (q, J = 7.6 Hz, 2H), 2.32 (s, 3H), 1.28 (t, J = 7.6 Hz, 3H).
c) To a solution of 3-ethyl-4-hydroxy-5-methyl-benzaldehyde (34.9 g, 0.213 mol) in MeCN (350 mL), K₂CO₃ (58.7 g, 0.425 mol) and benzylbromide (36.4 g, 0.213 mol) is added. The mixture is stirred at 60°C for 2 h before it is cooled to rt, diluted with water and extracted twice with EA. The org. extracts are washed with water and concentrated to give crude 4-benzyloxy-3-ethyl-5-methyl-benzaldehyde (45 g) as an orange oil. ¹H NMR (CDCl₃): *δ* 1.29 (t, *J* = 7.5 Hz, 3 H), 2.40 (s, 3 H), 2.77 (q, *J* = 7.8 Hz, 2 H), 4.90 (s, 2 H), 7.31-7.52 (m, 5 H), 7.62 (d, *J* = 1.5 Hz, 1 H), 7.66 (d, *J* = 1.8 Hz, 1 H), 9.94 (s, 1 H).
d) To a mixture of 4-benzyloxy-3-ethyl-5-methyl-benzaldehyde (132 g, 0.519 mol) and 2-methyl-2-butene (364 g, 5.19 mol) in tert.-butanol (1500 mL), a solution of NaH₂PO₄ dihydrate (249 g, 2.08 mol) in water (1500 mL) is added. To this mixture, NaClO₂ (187.8 g, 2.08 mol) is added in portions. The temperature of the reaction mixture is keept below 30°C, and evolution of gas is observed. Upon completion of the addition, the orange bi-phasic mixture is stirred well for 3 h before it is diluted with TBME (1500 mL). The org. layer is separated and washed with 20% aq. NaHS solution (1500 mL) and water (500 mL). The org. phase is then extracted three times with 0.5 N aq. NaOH (1000 mL), the aq. phase is acidified with 25 % aq. HCl (500 mL) and extracted twice with TBME (1000 mL). These org. extracts are combined and evparoated to dryness to give 4-benzyloxy-3-ethyl-5-methylbenzoic acid; ¹H NMR (D₆-DMSO): *δ* 1.17 (t, *J* = 7.5 Hz, 3 H), 2.31 (s, 3 H), 2.67 (q, *J* = 7.5 Hz, 2 H), 4.86 (s, 2 H), 7.34-7.53 (m, 5 H), 7.68 (s, 2 H), 12.70 (s, 1 H).

### 4-Benzyloxy-3-ethyl-5-methyl-benzoic acid hydrazide

To a solution of 4-benzyloxy-3-ethyl-5-methyl-benzoic acid (8.3 g, 30.7 mmol) in DCM (300 mL) is added DIPEA (10.7 mL) and the mixture is cooled to 0°C before PyBOP (14.5 g, 33.8 mmol) is added. After 10 min, a solution of 1M hydrazine in THF (100 mL) is added dropwise and the mixture is slowly warmed to rt during 2 h. The reaction mixture is then washed with sat. aq. NaHCO₃ followed by brine. The org. phase is collected, dried over MgSO₄, filtered and evaporated to give the title compound (24 g, 40% purity) as a yellow wax; LC-MS: t_{R} = 0.82 min; [M+H]⁺ = 285.10.

### Reference Example A

### 3-(2-Ethyl-4-{5-[6-(isopropyl-methyl-amino)-5-methyl-pyridin-3-yl]-[1,2,4]oxadiazol-3-yl}-6-methyl-phenyl)-propionic acid

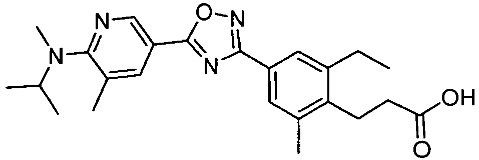

3-(2-Ethyl-4-{5-[6-(isopropyl-methyl-amino)-5-methyl-pyridin-3-yl]-[1,2,4]oxadiazol-3-yl}-6-methyl-phenyl)-propionic acid tert-butyl ester is prepared from 3-[2-ethyl-4-(N-hydroxycarbamimidoyl)-6-methyl-phenyl]-propionic acid tert-butyl ester and 6-(isopropyl-methyl-amino)-5-methyl-nicotinic acid in analogy to Example 1 below; LC-MS: t_{R} = 1.22 min; [M+1]⁺ = 479.33. This tert.butyl ester derivative is then treated with 6 N aq. HCl at 65°C for 18 h to give the title compound as a white solid; LC-MS: t_{R} = 1.02 min; [M+1]⁺ = 423.27; ¹H NMR (D₆-DMSO): *δ* 1.17-1.26 (m, 9 H), 2.37-2.43 (m, 8 H), 2.72 (q, J = 7.5 Hz, 2 H), 2.88 (s, 3 H), 2.91-2.99 (m, 2 H), 4.20-4.29 (m, 1 H), 7.72 (s, 1 H), 7.73 (s, 1 H), 8.11 (d, *J* = 1.8 Hz), 8.76 (d, *J* = 2.3 Hz, 1 H).

### Reference Example B

### 3-{2-Ethyl-6-methyl-4-[5-(5-methyl-6-pyrrolidin-1-yl-pyridin-3-yl)-[1,2,4]oxadiazol-3-yl]-phenyl}-propionic acid

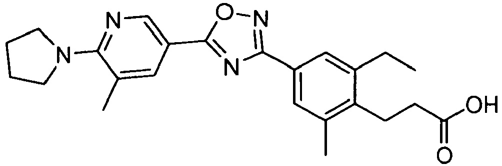

The title compound is prepared in analogy to Reference Example A starting from 5-methyl-6-pyrrolidin-1-yl-nicotinic acid; LC-MS: t_{R} = 0.86 min; [M+1]⁺ = 421.22.

### Reference Example C

### 3-{4-[5-(6-Diethylamino-5-ethyl-pyridin-3-yl)-[1,2,4]oxadiazol-3-yl]-2-ethyl-6-methylphenyl}-propionic acid

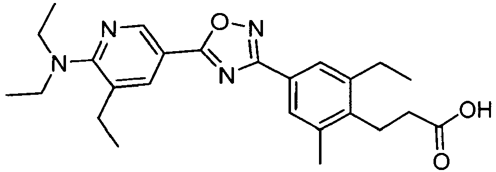

The title compound is prepared in analogy to Reference Example A starting from 6-diethylamino-5-ethyl-nicotinic acid; LC-MS: t_{R} =1.06 min; [M+1]⁺ = 437.29.

### Reference Example D

### 3-(2-Ethyl-4-{5-[5-ethyl-6-(isopropyl-methyl-amino)-pyridin-3-yl]-[1,2,4]oxadiazol-3-yl}-6-methyl-phenyl)-propionic acid

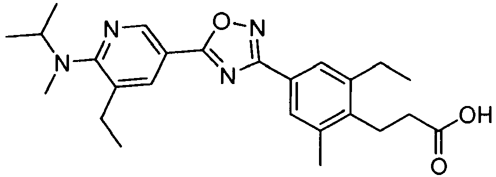

The title compound is prepared in analogy to Reference Example A starting from 5-ethyl-6-(isopropyl-methyl-amino)-nicotinic acid; LC-MS: t_{R} = 1.07 min; [M+1]⁺ = 437.26.

### Example 1

### 2-Ethyl-4-{5-[6-(ethyl-methyl-amino)-5-methyl-pyridin-3-yl]-[1,2,4]oxadiazol-3-yl}-6-methyl-phenol

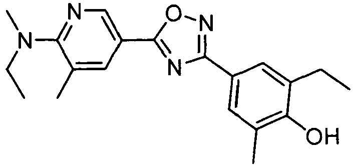

To a solution of 6-(ethyl-methyl-amino)-5-methyl-nicotinic acid (762 mg, 3.30 mmol) and NEt₃ (1.17 g, 11.6 mmol) in DMF (50 mL), TBTU (706 mg, 3.63 mmol) is added and the mixture is stirred at rt for 5 min before a solution of 3-ethyl-4,N-dihydroxy-5-methyl-benzamidine (642 mg, 3.30 mmol) in DMF (5 mL) is added. The mixture is stirred at rt for 16 h before another portion of TBTU (71 mg, 0.36 mmol) and 3-ethyl-4,N-dihydroxy-5-methyl-benzamidine (64 mg, 0.33 mmol) is added. Stirring is continued for 2 h. The mixture is diluted with EA (200 mL) and washed with sat. aq. NaHCO₃-solution (2x50 mL) followed by brine (50 mL): The organic extract is dried over Na₂SO₄, filtered and concentrated to give the crude hydroxyamidine ester intermediate; LC-MS: t_{R} = 0.76 min; [M+1]⁺ = 371.11. This material is dissolved in dioxane (50 mL) and the resulting solution is stirred at 95°C for 72 h. The solvent is removed in vacuo and the crude product is purified by prep. MPLC on silica gel eluting with a gradient of EA in heptane to give the title compound (868 mg) as a beige solid; LC-MS: t_{R} = 0.99 min; [M+1]⁺ = 353.13; ¹H NMR (CDCl₃): *δ* 1.26 (t, *J* = 7.0 Hz, 3 H), 1.33 (t, *J* = 7.3 Hz, 3 H), 2.36 (s, 3 H), 2.40 (s, 3 H), 2.73 (q, *J* = 7.5 Hz, 2 H), 3.06 (s, 3 H), 3.44 (q, *J* = 6.8 Hz, 2 H), 4.96 (s, 1 H), 7.84 (s, 2 H), 8.07 (s, 1 H), 8.89 (s, 1 H).

### Example 2

### (R)-3-(2-Ethyl-4-{5-[6-(ethyl-methyl-amino)-5-methyl-pyridin-3-yl]-[1,2,4]oxadiazol-3-yl}-6-methyl-phenoxy)-propane-1,2-diol

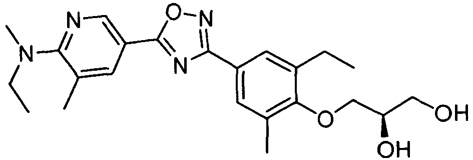

To a solution of 2-ethyl-4-(5-[6-(ethyl-methyl-aminoy)-methyl-pyridin-3-yl]-[1,2,4]oxadiazol-3-yl}-6-methyl-phenol (150 mg, 0.426 mmol) in isopropanol (5 mL) and 3 N aq. NaOH solution (1 mL), (R)3-chloro-1,2-propanediol (940 mg, 8.5 mmol) is added. The mixture is stirred at 60°C for 16 h before another portion of 3 N aq. NaOH solution (1 mL), (R)-3-chloro-1,2-propanediol (940 mg, 8.5 mmol) is added. Stirring is continued at 60°C for 48 h. Another portion of 3 N aq. NaOH solution (1 mL), (R)-3-chloro-1,2-propanediol (940 mg, 8.5 mmol) is added and the mixture is stirred at 60°C for further 3 days. The mixture is diluted with EA (100 mL) and washed with 1 N aq. NaOH solution (20 mL) followed by brine (20 mL). The org. extract is dried over MgSO₄, filtered and concentrated. The crude product is purified by prep. MPLC on silica gel eluting with a gradient of EA in heptane to give the title compound (80 mg) as a white solid; LC-MS: t_{R} = 0.89 min; [M+1]⁺ = 427.16; ¹H NMR (CDCl₃): *δ* 1.26 (t, *J* = 7.0 Hz, 3 H), 1.31 (t, *J* = 7.5 Hz, 3 H), 2.34 (s br, 1 H), 2.39 (s, 6 H), 2.75 (q, *J* = 7.3 Hz, 2 H), 2.92 (s br, 1 H), 3.05 (s, 3 H), 3.43 (q, *J* = 7.0 Hz, 2 H), 3.79-4.02 (m, 4 H), 4.11-4.21 (m, 1 H), 7.85 (s, 1 H), 7.87 (s, 1 H), 8.05 (s, 1 H), 8.88 (s, 1 H).

### Example 3

### (S)-3-(2-Ethyl-4-{5-[6-(ethyl-methyl-amino)-5-methyl-pyridin-3-yl]-[1,2,4]oxadiazol-3-yl}-6-methyl-phenoxy)-propane-1,2-diol

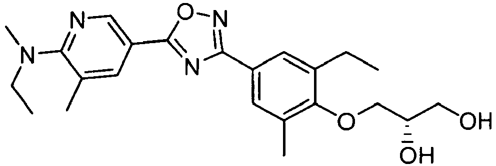

The title compound is prepared in analogy to Example 2 using (S)-3-chloro-1,2-propandiol; LC-MS: t_{R} = 0.89 min; [M+1]⁺ = 427.15.

### Example 4

### (R)-1-Amino-3-(2-ethyl-4-{5-[6-(ethyl-methyl-amino)-5-methyl-pyridin-3-yl]-[1,2,4]oxadiazol-3-yl}-6-methyl-phenoxy)-propan-2-ol

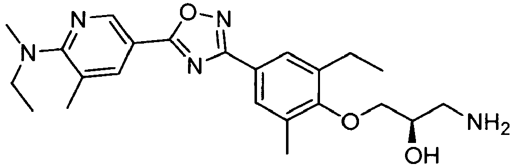

a) To a solution of 2-ethyl-4-(5-[6-(ethyl-methyl-amino)-methyl-pyridin-3-yl]-[1,2,4]oxadiazol-3-yl}-6-methyl-phenol (150 mg, 0.426 mmol) in isopropanol (20 mL) and 3 N aq. NaOH solution (5 mL), (S)-epichlorohydrine (525 mg, 5.68 mmol) is added. The mixture is stirred at rt for 16 h before another portion of (S)-epichlorohydrine (525 mg, 5.68 mmol) is added and stirring is continued for 24 h. The mixture is diluted with EA (200 mL) and washed with 1 N aq. NaOH solution (2x25 mL) and brine. The organic extract is dried over MgSO₄, filtered and concentrated to give crude ethyl-{5-[3-(3-ethyl-5-methyl-4-oxiranylmethoxy-phenyl)[1,2,4]oxadiazol-5-yl]-3-methyl-pyridin-2-yl}methyl-amine (271 mg); LC-MS: t_{R} = 1.08 min; [M+1]⁺ = 409.15.
b) The above epoxide (271 mg, 0.56 mmol) is dissolved in 7 N NH₃ in methanol ( 6 mL) and the resulting solution is stirred in a sealed vessel at 60°C for 6 h. The mixture is concentrated to give the title compound as a yellow oil; LC-MS: t_{R} = 0.77 min; [M+1]⁺ = 426.12.

### Example 5

### (S)-1-Amino-3-(2-ethyl-4-{5-[6-(ethyl-methyl-amino)-5-methyl-pyridin-3-yl]-[1,2,4]oxadiazol-3-yl}-6-methyl-phenoxy)-propan-2-ol

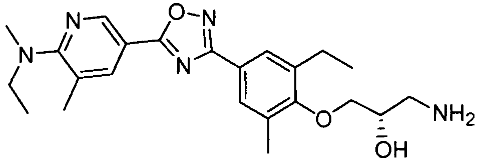

The title compound is prepared in analogy to Example 4 using (R)-epichlorohydrine; LC-MS: t_{R} = 0.77 min; [M+1]⁺ = 426.16.

### Example 6

### N-[(R)-3-(2-Ethyl-4-{5-[6-(ethyl-methyl-amino)-5-methyl-pyridin-3-yl]-[1,2,4]oxadiazol-3-yl}-6-methyl-phenoxy)-2-hydroxy-propyl)-2-hydroxy-acetamide

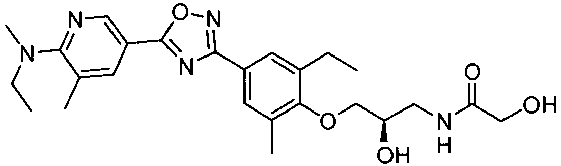

A solution of (R)-1-amino-3-(2-ethyl-4-{5-[6-(ethyl-methyl-amino)-5-methyl-pyridin-3-yl]-[1,2,4]oxadiazol-3-yl}-6-methyl-phenoxy)-propan-2-ol (238 mg, 0.560 mmol), glycolic acid (51 mg, 0.672 mmol), DIPEA (290 mg, 2.24 mmol) and HOBT (95 mg, 0.70 mmol) in THF (20 mL) is stirred at rt for 15 min before EDC HCl (134 mg, 0.70 mmol) is added. The mixture is stirred at rt for 16 h before it is diluted with EA 8100 mL) and washed with 1 N aq. NaOH solution (3x25 mL) and brine (25 mL). The organic extract is dried over Na₂SO₄, filtered and concentrated. The crude product is purified by prep. HPLC on silica gel eluting with a gradient of EA in heptane to give the title compound (34 mg) as a colourless resin; LC-MS: t_{R} = 0.85 min; [M+1]⁺ = 484.31; ¹H NMR (CD₃OD): *δ*1.26 (t, *J* = 7.0 Hz, 3 H), 1.31 (t, *J* = 7.5 Hz, 3 H), 2.41 (s, 3 H), 2.43 (s, 3 H), 2.80 (q, *J* = 7.3 Hz, 2 H), 3.07 (s, 3 H), 3.44-3.52 (m, 3 H), 3.66 (dd, *J* = 13.0, 4.3 Hz, 1 H), 3.85-3.91 (m, 2 H), 4.04 (s, 2 H), 4.12-4.18 (m, 1 H), 7.82 (s, 1 H), 7.85 (s, 1 H), 8.12 (s, 1 H), 8.81 (s, 1 H).

### Example 7

### N-[(S)-3-(2-Ethyl-4-(5-[6-(ethyl-methyl-amino)-5-methyl-pyridin-3-yl]-[1,2,4]oxadiazol-3-yl}-6-methyl-phenoxy)-2-hydroxy-propyl]-2-hydroxy-acetamide

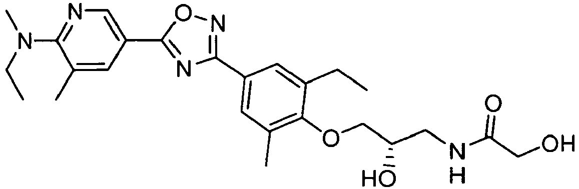

The title compound is prepared in analogy to Example 6 starting from Example 5; LC-MS: t_{R} = 0.85 min; [M+1]⁺ = 484.30.

### Example 8

### N-((S)-3-{4-[5-(6-Diethylamino-5-methyl-pyridin-3-yl)-[1,2,4]oxadiazol-3-yl]-2-ethyl-6-methyl-phenoxy}-2-hydroxy-propyl)-2-hydroxy-acetamide

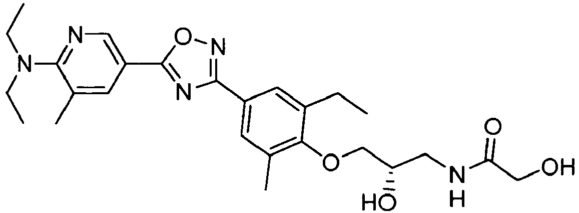

To a solution of 6-diethylamino-5-methyl-nicotinic acid (88 mg, 0.36 mmol) and HOBT (53 mg, 0.396 mmol) in THF (5 mL), EDC HCl (76 mg, 0.396 mmol) is added. The mixture is stirred at rt for 5 min before N-((S)-3-[2-ethyl-4-(N-hydroxycarbamimidoyl)-6-methyl-phenoxy]-2-hydroxy-propyl)-2-hydroxy-acetamide 129 mg, 0.396 mmol) is added. The reaction is stirred at rt for 18 h. The mixture is diluted with sat. aq. NaHCO₃ solution and extracted twice with EA. The combined organic extracts are dried over MgSO₄, filtered and evaporated to give the crude hydroxyamidine ester intermediate; LC-MS: t_{R} = 0.71 min; [M+1]⁺ = 516.33. This intermediate is dissolved in dioxane (10 mL) and the mixture is stirred at 80°C for 16 h. The solvent is evaporated and the crude product is purified on prep. TLC using DCM containing 10% of methanol to give the title compound (38 mg) as a colourless resin; LC-MS: t_{R} = 0.88 min; [M+1]⁺ = 498.27;

### Example 9

### 2-Ethyl-4-{5-[6-(isopropyl-methyl-amino)-5-methyl-pyridin-3-yl]-(1,2,4]oxadiazol-3-yl}-6-methyl-phenol

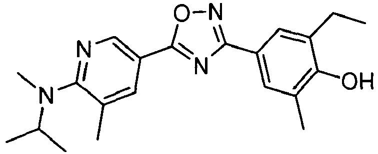

The title compound is prepared in analogy to Example 1 starting from 6-(isopropyl-methylamino)-5-methyl-nicotinic acid; LC-MS: t_{R} = 1.03 min; [M+1]⁺ = 367.23; ¹H NMR (CDCl₃): *δ* 1.26 (d, *J* = 6.5 Hz, 6 H), 1.33 (t, *J* = 7.5 Hz, 3 H), 2.36 (s, 3 H), 2.39 (s, 3 H), 2.73 (q, *J* = 7.5 Hz, 2 H), 2.91 (s, 3 H), 4.13-4.21 (m, 1 H), 4.96 (s, 1 H), 7.83 (s, 2 H), 8.08 (d, *J* = 1.3 Hz, 1 H), 8.90 (d, *J* = 2.0 Hz, 1 H).

### Examples 10 to 15

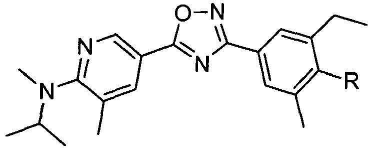

The following Examples are prepared starting from Example 9 in analogy to previous Examples.

| **Example** | **prepared in analogy to Example** | **R** | **LC-MS t_{R} (min) [M+H]⁺** | |
|---|---|---|---|---|
| 10 | 2 | 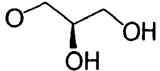 | 0.92 | 441.26 |
| 11 | 2 | 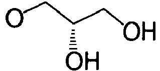 | 0.92 | 441.26 |
| 12 | 4 | 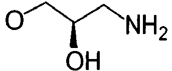 | 0.78 | 440.29 |
| 13 | 4 | 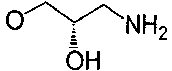 | 0.78 | 440.28 |
| 14 | 6 | 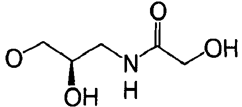 | 0.88 | 498.28 |
| 15 | 6 | 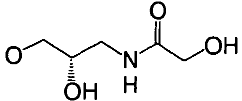 | 0.88 | 498.30 |

### Example 15

¹H NMR (CDCl₃): *δ*1.26 (d, *J* = 6.8 Hz, 6 H), 1.32 (t, *J* = 7.5 Hz, 3 H), 2.39 (s, 6 H), 2.57 (s br, 1 H), 2.75 (q, *J* = 7.5 Hz, 2 H), 2.91 (s, 3 H), 3.33 (s br, 1 H), 3.48-3.57 (m, 1 H), 3.77-3.86 (m, 2 H), 3.90 (m, 1 H), 4.14-4.25 (m, 4 H), 6.99 (t br, *J* = 6.3 Hz, 1 H), 7.85 (s, 1 H), 7.87 (s, 1 H), 8.07 (d, J = 1.5 Hz, 1 H), 8.90 (d, *J* = 2.0 Hz, 1 H).

### Example 16

### 2-Ethyl-6-methyl-4-[5-(5-methyl-6-pyrrolidin-1-yl-pyridin-3-yl)-[1,2,4]oxadiazol-3-yl]-phenol

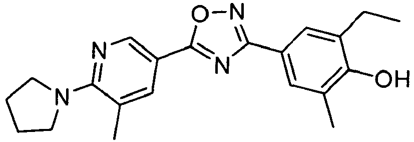

The title compound is prepared in analogy to Example 1 starting from 5-methyl-6-pyrrolidin-1-yl-nicotinic acid; LC-MS: t_{R} = 0.87 min; [M+1]⁺ = 365.22.

### Examples 17 to 21

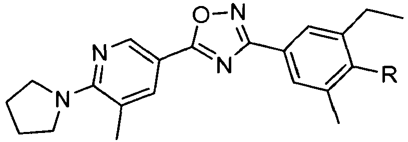

The following Examples are prepared starting from Example 16 in analogy to previous Examples.

| **Example** | **prepared in analogy to Example** | **R** | **LC-MS t_{R} (min) [M+H]⁺** | |
|---|---|---|---|---|
| 17 | 2 | 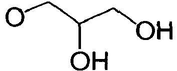 | | |
| 18 | 4 | 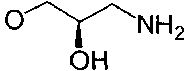 | 0.70 | 438.24 |
| 19 | 4 | 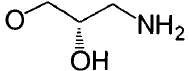 | 0.70 | 438.24 |
| 20 | 6 | 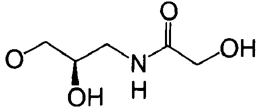 | 0.76 | 496.30 |
| 21 | 6 | 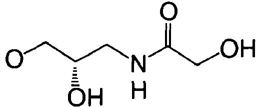 | 0.76 | 496.29 |

### Example 21

¹H NMR (CDCl₃): *δ*1.31 (t, *J* = 7.5 Hz, 3 H), 1.97-2.03 (m, 4 H), 2.38 (s, 3 H), 2.49 (s, 3 H), 2.74 (q, *J* = 7.5 Hz, 2 H), 3.41 (d, *J* = 4.3 Hz, 1 H), 3.48-3.56 (m, 1 H), 3.71-3.77 (m, 4 H), 3.77-3.92 (m, 3 H), 4.17-4.24 (m, 3 H), 7.02 (t br, *J* = 6.0 Hz, 1 H), 7.84 (s, 1 H), 7.86 (s, 1 H), 7.96 (d, *J* = 1.3 Hz, 1 H), 8.82 (d, *J* = 2.3 Hz, 1 H).

### Example 22

### 4-[5-(6-Diethylamino-5-ethyl-pyridin-3-yl)-[1,2,4]oxadiazol-3-yl]-2-ethyl-6-methyl-phenol

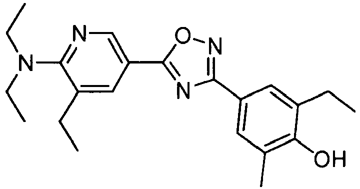

The title compound is prepared in analogy to Example 1 starting from 6-diethylamino-5-ethyl-nicotinic acid; LC-MS: t_{R} = 1.07 min; [M+1]⁺ = 381.29.

### Examples 23 to 28

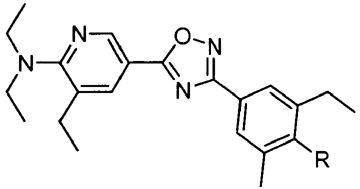

The following Examples are prepared starting from Example 22 in analogy to previous Examples.

| **Example** | **prepared in analogy to Example** | **R** | **LC-MS t_{R} (min) [M+H]⁺** | |
|---|---|---|---|---|
| 23 | 2 | 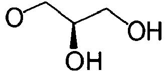 | 0.97 | 455.30 |
| 24 | 2 | 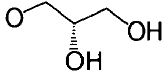 | 0.98 | 455.30 |
| 25 | 4 | 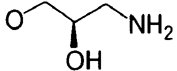 | 0.82 | 454.29 |
| 26 | 4 | 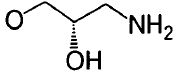 | 0.82 | 454.31 |
| 27 | 6 | 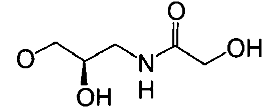 | 0.93 | 512.37 |
| 28 | 6 | 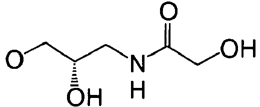 | 0.92 | 512.37 |

### Example 28

¹H NMR (CDCl₃): *δ*1.20 (t, *J* = 7.0 Hz, 6 H), 1.29-1.37 (m, 6 H), 2.39 (s, 3 H), 2.51 (t, *J* = 5.0 Hz, 1 H), 2.68-2.79 (m, 4 H), 3.32 (d, *J* = 4.3 Hz, 1 H), 3.43 (q, *J* = 7.0 Hz, 4 H), 3.49-3.57 (m, 1 H), 3.77-3.87 (m, 2 H), 3.88-3.93 (m, 1 H), 4.19-4.24 (m, 3 H), 6.97 (t br, *J* = 6.5 Hz, 1 H), 7.87 (s, 1 H), 7.88 (s, 1 H), 8.14 (s br, 1 H), 8.92 (d, *J* = 1.8 Hz, 1 H).

### Example 29

### 2-Ethyl-4-{5-[5-ethyl-6-(isopropyl-methyl-amino)-pyridin-3-yl]-[1,2,4]oxadiazol-3-yl}-6-methyl-phenol

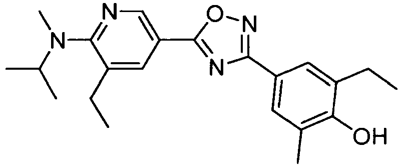

The title compound is prepared in analogy to Example 1 starting from 5-ethyl-6-(isopropyl-methyl-amino)-nicotinic acid; LC-MS: t_{R} = 1.08 min; [M+1]⁺ = 381.28.

### Examples 30 to 35

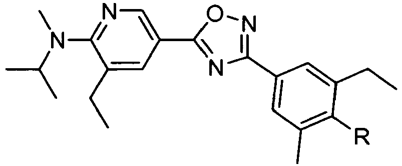

The following Examples are prepared starting from Example 29 in analogy to previous Examples.

| **Example** | **prepared in analogy to Example** | **R** | **LC-MS t_{R} (min) [M+H]⁺** | |
|---|---|---|---|---|
| 30 | 2 | 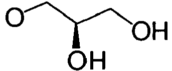 | 0.98 | 455.30 |
| 31 | 2 | 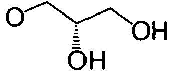 | 0.98 | 455.30 |
| 32 | 4 | 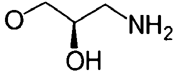 | | |
| 33 | 4 | 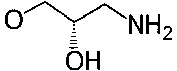 | 0.82 | 454.32 |
| 34 | 6 | 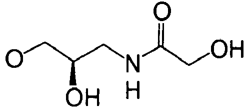 | 0.93 | 512.38 |
| 35 | 6 | 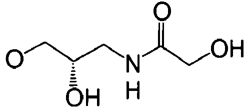 | 0.94 | 512.38 |

### Example 31

¹H NMR (CDCl₃): *δ*1.25 (d, *J* = 6.8 Hz, 6 H), 1.33 (t, *J* = 7.5 Hz, 6 H), 2.05 (t, *J* = 5.8 Hz, 1 H), 2.41 (s, 3 H), 2.70-2.81 (m, 5 H), 2.89 (s, 3 H), 3.82-3.92 (m, 2 H), 3.94-3.98 (m, 2 H), 4.09-4.20 (m, 2 H), 7.88 (s, 1 H), 7.89 (s, 1 H), 8.15 (d, *J* = 1.5 Hz, 1 H), 8.91 (d, *J* = 1.8 Hz, 1H).

### Example 36

### 2-Ethyl-4-[5-(5-ethyl-6-pyrrolidin-1-yl-pyridin-3-yl)-[1,2,4]oxadiazol-3-yl]-6-methylphenol

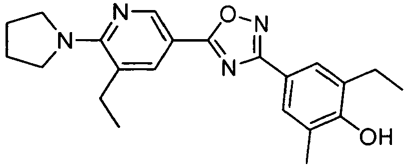

The title compound (791 mg) is obtained as an off-white solid in analogy to Example 1 starting from 5-ethyl-6-pyrrolidin-1-yl-nicotinic acid (1.80 g, 8.17 mmol) and 3-ethyl-4,N-dihydroxy-5-methyl-benzamidine (1.59 g, 8.17 mmol); LC-MS: t_{R} = 0.91 min; [M+1]⁺= 379.29; ¹H NMR (CDCl₃): *δ*1.27 (t, *J=* 7.5 Hz, 3 H), 1.33 (t, *J=* 7.5 Hz, 3 H), 1.98-2.05 (m, 4 H), 2.36 (s, 3 H), 2.73 (q, *J* = 7.8 Hz, 2 H), 2.82 (q, *J* = 7.3 Hz, 2 H), 3.67-3.73 (m, 4 H), 4.96 (s, 1H), 7.84 (s, 2 H), 8.04 (d, *J* = 2.3 Hz, 1 H), 8.85 (d, *J* = 2.3 Hz, 1 H).

### Example 37

### 3-(2-Ethyl-4-{5-(6-(isopropyl-methyl-amino)-5-methyl-pyridin-3-yl]-(1,2,4]oxadiazol-3-yl}-6-methyl-phenyl)-N-(2-hydroxy-ethyl)-propionamide

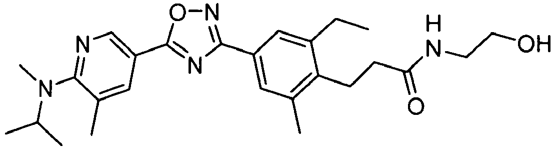

To a solution of 3-(2-ethyl-4-{5-[6-(isopropyl-methyl-amino)-5-methyl-pyridin-3-yl]-[1,2,4]oxadiazol-3-yl}-6-methyl-phenyl-propionic acid (73 mg, 0.172 mmol) and DIPEA (67 mg, 0.515 mmol) in DMF (5 mL), PyBOP (117 mg, 0.189 mmol) is added. The mixture is stirred for 15 min at rt before ethanolamine (42 mg, 0.682 mmol) is added and stirring is continued for 15 h at rt. The reaction is quenched by adding water and sat. aq. NaHCO₃-solution and the mixture is extracted twice with EA. The combined organic extracts are dried over MgSO₄, filtered and concentrated. The obtained solid is washed with ether, then further purified by prep. HPLC to give the title compound (14 mg) as a white solid; LC-MS: t_{R} = 0.92 min; [M+1]⁺ = 466.26; ¹H NMR (CDCl3): *δ*1.27 (d, *J* = 6.8 Hz, 6 H), 1.32 (t, *J* = 7.5 Hz, 3 H), 2.40 (s, 3 H), 2.40-2.45 (m, 2 H), 2.46 (s, 3 H), 2.78 (q, *J* = 7.5 Hz, 2 H), 2.92 (s, 3 H), 3.08-3.14 (m, 2 H), 3.46 (q, *J* = 5.3 Hz, 2 H), 3.73-3.78 (m, 2 H), 4.15-4.23 (m, 1 H), 5.82 (t br, *J* = 5.0 Hz, 1 H), 7.83 (s, 1 H), 7.85 (s, 1 H), 8.09 (d, *J* = 1.0 Hz, 1 H), 8.91 (d, *J* = 1.0 Hz, 1 H).

### Example 38

### [3-(2-Ethyl-4-{5-[6-(isopropyl-methyl-amino)-5-methyl-pyridin-3-yl]-[1,2,4]oxadiazol-3-yl}-6-methyl-phenyl)-propionylamino]-acetic acid

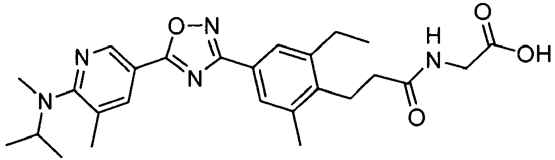

The title compound is prepared in analogy to Example 37 by coupling glycine to 3-(2-ethyl-4-{5-[6-(isopropyl-methyl-amino)-5-methyl-pyridin-3-yl]-[1,2,4]oxadiazol-3-yl}-6-methyl-phenyl)-propionic acid; LC-MS: t_{R} = 0.93 min; [M+1]⁺ = 480.29; ¹H NMR (CDCl₃): *δ*1.26 (d, *J* = 6.5 Hz, 6 H), 1.31 (t, *J* = 7.5 Hz, 3 H), 2.39 (s, 3 H), 2.43-2.50 (m, 5 H), 2.77 (q, *J* = 7.5 Hz, 2 H), 2.91 (s, 3 H), 3.07-3.14 (m, 2 H), 4.11-4.20 (m, 3 H), 6.04 (t br, *J* = 4.3 Hz, 1 H), 7.82 (s, 1 H), 7.84 (s, 1 H), 8.09 (s, 1 H), 8.92 (d, *J* = 1.8 Hz, 1 H).

### Example 39

### 1-[3-(2-Ethyl-4-{5-(6-(isopropyl-methyl-amino)-5-methyl-pyridin-3-yl]-[1,2,4]oxadiazol-3-yl}-6-methyl-phenyl)-propionyl]-azetidine-3-carboxylic acid

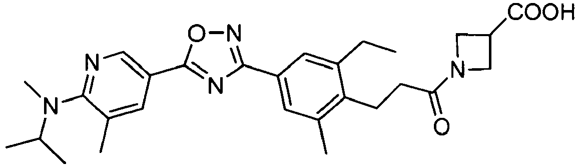

The title compound is prepared in analogy to Example 37 by coupling azetidine-3-carboxylic acid to 3-(2-ethyl-4-{5-[6-(isopropyl-methyl-amino)-5-methyl-pyridin-3-yl]-[1,2,4]oxadiazol-3-yl}-6-methyl-phenyl)propionic acid; LC-MS: t_{R} = 0.94 min; [M+1]⁺ = 506.31; ¹H NMR (CDCl₃): *δ*1.26 (d, *J* = 6.8 Hz, 6 H), 1.31 (t, *J* = 7.5 Hz, 3 H), 2.27-2.34 (m, 2 H), 2.39 (s, 3 H), 2.45 (s, 3 H), 2.77 (q, *J* = 7.3 Hz, 2 H), 2.91 (s, 3 H), 3.05-3.12 (m, 2 H), 3.35-3.44 (m, 1 H), 4.01-4.07 (m, 1 H), 4.10-4.20 (m, 2 H), 4.20-4.27 (m, 2 H), 7.81 (s, 1 H), 7.83 (s, 1 H), 8.08 (d, *J* = 1.0 Hz, 1 H), 8.90 (d, *J =* 2.0 Hz, 1 H).

### Examples 40 to 42

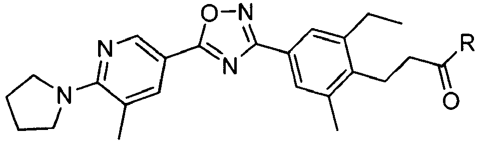

The following Examples are prepared in analogy to previous Examples starting from Reference Example B.

| **Example** | **R** | **in analogy to Example** | **LC-MS t_{R} [min] [M+H]⁺** | |
|---|---|---|---|---|
| 40 | 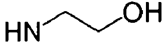 | 37 | 0.79 | 464.27 |
| 41 | 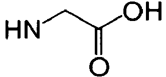 | 38 | 0.80 | 478.30 |
| 42 | 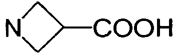 | 39 | 0.78 | 504.36 |

### Examples 43 to 45

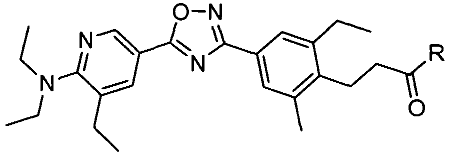

The following Examples are prepared in analogy to previous Examples starting from Reference Example C.

| **Example** | **R** | **in analogy to Example** | **LC-MS t_{R} [min] [M+H]⁺** | |
|---|---|---|---|---|
| 43 | 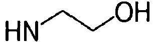 | 37 | 0.97 | 480.34 |
| 44 | 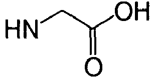 | 38 | 0.97 | 494.33 |
| 45 | 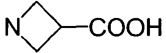 | 39 | 0.98 | 520.37 |

### Example 44

¹H NMR *δ*(CDCl₃): 1.20 (t, *J =* 6.8 Hz, 6 H), 1.33 (q, *J =* 7.5 Hz, 6 H), 2.45 (s, 3 H), 2.46-2.50 (m, 2 H), 2.69-2.81 (m, 4 H), 3.08-3.14 (m, 2 H), 3.44 (q, *J* = 7.0 Hz, 4 H), 4.14-4.18 (m, 2 H), 6.03 (t br, *J* = 4.8 Hz, 1 H), 7.83 (s, 1 H), 7.85 (s, 1 H), 8.16 (d, *J* = 0.8 Hz, 1 H), 8.94 (d, *J* = 0.5 Hz, 1 H).

### Examples 46 to 48

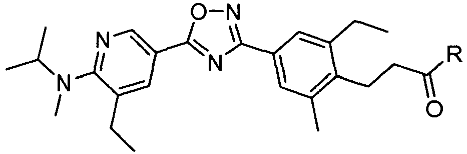

The following Examples are prepared in analogy to previous Examples starting from Reference Example D.

| **Example** | **R** | **in analogy to Example** | **LC-MS t_{R} [min] [M+H]⁺** | |
|---|---|---|---|---|
| 46 | 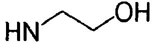 | 37 | 0.98 | 480.34 |
| 47 | 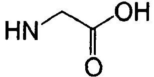 | 38 | 0.98 | 494.34 |
| 48 | 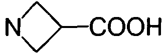 | 39 | 0.99 | 520.38 |

### Example 46

¹H NMR (CDCl₃): *δ*1.25 (d, *J* = 6.5 Hz, 6 H), 1.29-1.36 (m, 6 H), 2.39-2.45 (m, 2 H), 2.46 (s, 3 H), 2.73 (q, *J* = 7.5 Hz, 2 H), 2.78 (q, *J* = 7.5 Hz, 2 H), 2.89 (s, 3 H), 3.08-3.15 (m, 2 H), 3.46 (q, *J* = 5.3 Hz, 2 H), 3.72-3.79 (m, 2 H), 4.13 (hept, *J* = 6.8 Hz, 1 H), 5.83 (t br, *J* = 4.5 Hz, 1 H), 7.84 (s, 1 H), 7.86 (s, 1 H), 8.15 (d, *J* = 1.5 Hz, 1 H), 8.91 (d, *J* = 1.8 Hz, 1 H).

### Example 49

### (R)-3-{2-Ethyl-4-[5-(5-ethyl-6-pyrrolidin-1-yl-pyridin-3-yl)-[1,2,4]oxadiazol-3-yl]-6-methyl-phenoxy}-propane-1,2-diol

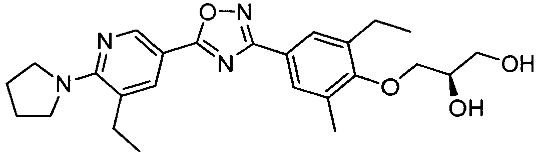

The title compound (97 mg) is obtained as a colourless oil starting from Example 36 (129 mg, 0.341 mmol) following the procedure given for Example 2; LC-MS: t_{R} = 0.81 min; [M+1]⁺= 453.09.

### Example 50

### (S)-3-{2-Ethyl-4-[5-(5-ethyl-6-pyrrolidin-1-yl-pyridin-3-yl)-[1,2,4]oxadiazol-3-yl]-6-methyl-phenoxy}-propane-1,2-diol

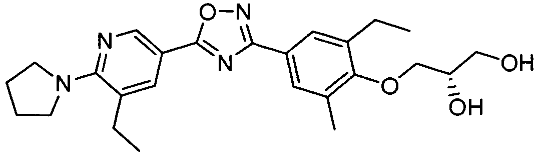

The title compound (117 mg) is obtained as a coloureless oil starting from Example 36 (132 mg, 0.348 mmol) following the procedure given for Example 3; LC-MS: t_{R} = 0.81 min; [M+1]⁺ = 453.10;¹H NMR (CDCl₃): *δ*1.27 (t, *J* = 7.5 Hz, 3 H), 1.32 (t, *J* = 7.5 Hz, 3 H), 1.98-2.04 (m, 4 H), 2.13 (s br, 1 H), 2.40 (s, 3 H), 2.72-2.87 (m, 5 H), 3.67-3.73 (m, 4 H), 3.83-3.99 (m, 4 H), 4.14-4.21 (m, 1 H), 7.86 (s, 1 H), 7.88 (s, 1 H), 8.03 (d, J = 2.0 Hz, 1 H), 8.85 (d, *J* = 2.0 Hz, 1 H).

### Example 51

### (R)-1-Amino-3-{2-ethyl-4-[5-(5-ethyl-6-pyrrolidin-1-yl-pyridin-3-yl)-[1,2,4]oxadiazol-3-yl]-6-methyl-phenoxy}-propan-2-ol

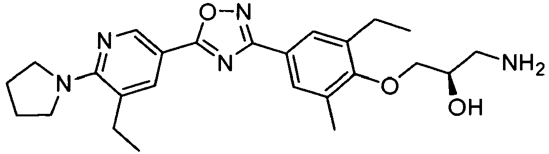

The title compound is prepared in analogy to Example 4 starting from Example 36; LC-MS: t_{R} = 0.72 min; [M+1]⁺ = 452.11.

### Example 52

### (S)-1-Amino-3-{2-ethyl-4-[5-(5-ethyl-6-pyrrolidin-1-yl-pyridin-3-yl)-[1,2,4]oxadiazol-3-yl]-6-methyl-phenoxy}-propan-2-ol

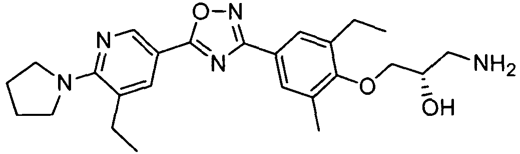

The title compound is prepared in analogy to Example 5 starting from Example 36; LC-MS: t_{R} = 0.72 min; [M+1]⁺= 452.13.

### Example 53

### N-((R)-3-{2-Ethyl-4-[5-(5-ethyl-6-pyrrolidin-1-yl-pyridin-3-yl)-[1,2,4]oxadiazol-3-yl]-6-methyl-phenoxy}-2-hydroxy-propyl)-2-hydroxy-acetamide

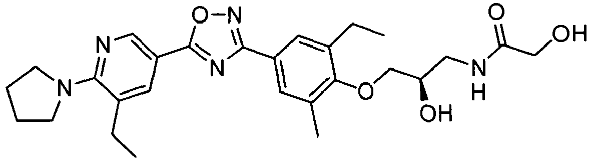

The title compound (110 mg) is obtained as a white solid in analogy to Example 6 starting from Example 52 (123 mg, 272 µmol); LC-MS: t_{R} = 0.78 min; [M+1]⁺ = 510.15; ¹H NMR (CDCl₃): *δ*1.27 (t, *J* = 7.8 Hz, 3 H), 1.31 (t, *J =* 7.5 Hz, 3 H), 1.97-2.05 (m, 4 H), 2.38 (s, 3 H), 2.74 (q, *J* = 7.5 Hz, 2 H), 2.82 (q, *J* = 7.5 Hz, 2 H), 2.93 (t br, *J* = 4.3 Hz, 1 H), 3.46-3.55 (m, 2 H), 3.67-3.73 (m, 4 H), 3.77-3.86 (m, 2 H), 3.89 (dd, *J* = 9.8, 4.8 Hz, 1 H), 4.17-4.23 (m, 3 H), 7.06 (t br, *J* = 6.0 Hz, 1 H), 7.84 (s, 1 H), 7.86 (s, 1 H), 8.03 (d, *J* = 2.3 Hz, 1 H), 8.84 (d, *J* = 2.3 Hz, 1 H).

### Example 54

### N-((S)-3-(2-Ethyl-4-[5-(5-ethyl-6-pyrrolidin-1-yl-pyridin-3-yl)-[1,2,4]oxadiazol-3-yl]-6-methyl-phenoxy}-2-hydroxy-propyl)-2-hydroxy-acetamide

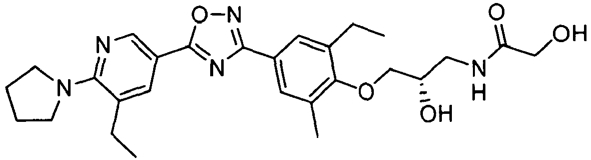

The title compound (110 mg) is obtained as a white solid in analogy to Example 6 starting from Example 53 (138 mg, 306 µmol); LC-MS: t_{R} = 0.78 min; [M+1]⁺ = 510.15.

### Examples 55 to 58

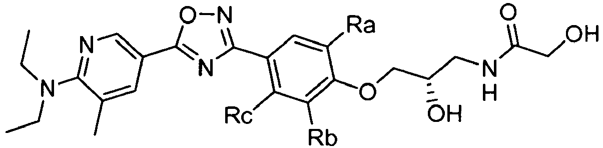

The following Examples were prepared in analogy to Example 8 starting from 6-diethylamino-5-methyl-nicotinic acid and the appropriate N-hydroxy-benzamidine.

| **Example** | **Rₐ** | **R_{b}** | **R_{c}** | **LC-MS** t_{R} [min] [M+H]⁺** | |
|---|---|---|---|---|---|
| 55 | CH₃ | CH₃ | H | 0.63 | 484.33 |
| 56 | CH₃ | CI | H | 0.66 | 504.17 |
| 57 | CH₃ | OCH₃ | H | 0.62 | 500.25 |
| 58 | H | H | CH₃ | 0.60 | 470.29 |

### Example 55

¹H NMR (CDCl₃): *δ*1.22 (t, *J* = 7.0 Hz, 6 H), 2.37 (s, 6 H), 2.38 (s, 3 H), 2.83 (t, J = 5.3 Hz, 1 H), 3.43-3.56 (m, 6 H), 3.76-3.86 (m, 2 H), 3.90 (dd, *J* = 9.5, 4.5 Hz, 1 H), 4.16-4.24 (m, 3 H), 7.05 (t, *J* = 5.8 Hz, 1 H), 7.84 (s, 2 H), 8.05 (d, *J* = 1.8 Hz, 1 H), 8.89 (d, *J* = 2.0 Hz, 1 H).

### Example 56

¹H NMR (CDCl₃): *δ*1.22 (t, *J* = 7.0 Hz, 6 H), 2.38 (s, 3 H), 2.42 (s, 3 H), 2.94 (s br, 1 H), 3.48 (q, *J* = 7.3 Hz, 4 H), 3.53-3.65 (m, 2 H), 3.81 (ddd, *J* = 14.1, 6.5, 3.5 Hz, 1 H), 3.99 (dd, *J* = 9.3, 6.0 Hz, 1 H), 4.05 (dd, *J* = 9.5, 4.8 Hz, 1 H), 4.18-4.26 (m, 3 H), 7.10 (t, *J* = 5.5 Hz, 1 H), 7.91 (d, *J* = 1.5 Hz, 1 H), 8.03 (d, *J* = 1.8 Hz, 1 H), 8.04 (d, *J* = 1.8 Hz, 1 H), 8.87 (d, *J* = 2.0 Hz, 1 H).

### Example 57

¹H NMR (CDCl₃): *δ*1.22 (t, *J* = 7.0 Hz, 6 H), 2.38 (s, 3 H), 2.39 (s, 3 H), 2.93 (t br, *J* = 5.3 Hz, 1 H), 3.48 (q, *J* = 7.0 Hz, 2 H), 3.51 (s, 3 H), 3.75 (ddd, *J* = 13.8, 6.5, 3.3 Hz, 1 H), 3.89-3.97 (m, 2 H), 3.98 (s, 3 H), 4.07-4.14 (m, 2 H), 4.18 (d, *J* = 4.8 Hz, 2 H), 7.04 (t br, *J* = 5.8 Hz, 1 H), 7.56 (d, *J* = 1.8 Hz, 1 H), 7.65 (d, *J* = 1.3 Hz, 1 H), 8.05 (d, *J* = 1.8 Hz, 1 H), 8.89 (d, *J* = 2.0 Hz, 1 H).

### Example 58

¹H NMR (CDCl₃): *δ*1.22 (t, *J =* 7.0 Hz, 6 H), 2.38 (s, 3 H), 2.66 (s, 3 H), 3.17 (s br, 1 H), 3.47 (q, *J* = 6.8 Hz, 4 H), 3.49-3.56 (m, 1 H), 3.69-3.78 (m, 1 H), 3.99-4.07 (m, 2 H), 4.15-4.22 (m, 3 H), 6.84-6.89 (m, 2 H), 7.10 (t, *J* = 6.0 Hz, 1 H), 8.00-8.07 (m, 2 H), 8.89 (d, *J* = 2.0 Hz, 1 H).

### Examples 59 to 60

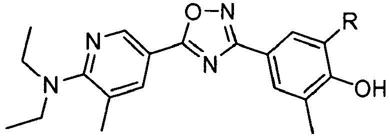

The following Examples were prepared in analogy to Example 8 starting from 6-diethylamino-5-methyl-nicotinic acid and the appropriate 4,N-dihydroxy-benzamidine.

| **Example** | **R** | **LC-MS t_{R} [min] [M+H]⁺** | |
|---|---|---|---|
| 59 | Cl | 0.78 | 373.24 |
| 60 | n-propyl | 0.80 | 381.22 |

### Example 60

¹H NMR (CDCl₃): *δ*1.04 (t,*J*=7.3 Hz, 3H), 1.22 (t, *J*=6.5Hz, 6H), 1.68-1.79 (m, 2 H), 2.35 (s, 3 H), 2.38 (s, 3 H), 2.68 (t, *J* = 7.5 Hz, 2 H), 3.47 (q, *J* = 6.8 Hz, 4 H), 4.99 (s, 1 H), 7.82 (s, 1 H), 7.83 (s, 1 H), 8.07 (s, 1 H), 8.90 (s, 1 H).

### Example 61

### (R)-3-{4-[5-(6-Diethylamino-5-methyl-pyridin-3-yl)-[1,2,4]oxadiazol-3-yl]-2,6-dimethyl-phenoxy}-propane-1,2-diol

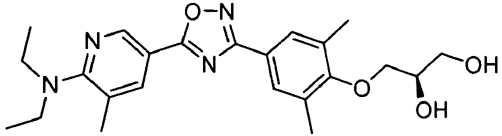

a) To solution of 6-diethylamino-5-methyl-nicotinic acid hydrochloride (120 mg, 490 µmol) in DMF, DIPEA (190 mg, 1.47 mmol) followed by TBTU (165 mg, 515 µmol) is added. The mixture is stirred at rt for 30 min before (S)-4-(2,2-Dimethyl-[1,3]dioxolan-4-ylmethoxy)-N-hydroxy-3,5-dimethyl-benzamidine (152 mg, 515 µmol) is added. The mixture is stirred at rt for 1 h before it is diluted with EA, washed with sat. aq. NaHCO₃ solution, dried over MgSO₄, filtered and concentrated. The residue is dissolved in dioxane (5 mL) and the resulting solution is stirred at 80°C for 16 h. The sovent is evaporated and the crude product is purified by prep. HPLC to give (5-{3-[4-(S)-(2,2-dimethyl-[1,3]dioxolan-4-ylmethoxy)-3,5-dimethylphenyl]-[1,2,4]oxadiazol-5-yl}-3-methyl-pyridin-2-yl)-diethyl-amine (90 mg) as a colourless oil; LC-MS: t_{R} = 0.88 min; [M+1]⁺ = 467.27.
b) The above acetal (90 mg, 193 µmol) is dissolved in 25% aq. HCl (3 mL) and dioxane (3 mL) and the mixture is stirred at rt for 24 h. The mixture is concentrated and the crude product is purified on prep. TLC using DCM:methanol 92:8 to give the title compound () as a white solid; LC-MS: t_{R} = 0.66 min; [M+1]⁺ = 427.26. ¹H NMR (CDCl₃): *δ*1.22 (t, *J* = 7.0 Hz, 6 H), 2.13 (s br, 1 H), 2.39 (s, 3 H), 2.39 (s, 6 H), 2.78 (d br, *J* = 3.5 Hz, 1 H), 3.48 (q, *J* = 7.0 Hz, 4 H), 3.82-3.97 (m, 4 H), 4.13-4.20 (m, 1 H), 7.85 (s, 2 H), 8.06 (d, *J* = 1.8 Hz, 1 H), 8.89 (d, *J* = 2.3 Hz, 1 H).

### Examples 62 to 64

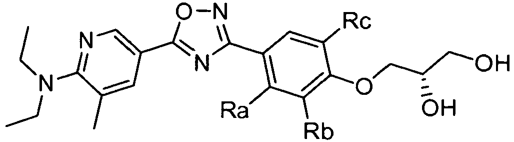

The following Examples are prepared in analogy to Example 61 using the appropriate hydroxy-benzamidines.

| **Example** | **Rₐ** | **R_{b}** | **R_{c}** | **LC-MS** t_{R} [min] [M+H]⁺** | |
|---|---|---|---|---|---|
| 62 | OCH₃ | H | H | 0.55 | 429.19 |
| 63 | H | CH₃ | Cl | 0.70 | 447.14 |
| 64 | H | OCH₃ | Cl | 0.69 | 463.16 |

### Example 63

¹H NMR (CDCl₃): *δ*1.22 (t, *J* = 6.8 Hz, 6 H), 2.11 (t br, *J*=5.3 Hz, 1 H), 2.39 (s, 3 H), 2.44 (s, 3 H), 2.86 (d br, *J* = 4.8 Hz, 1 H), 3.49 (q, *J* = 6.8 Hz, 4 H), 3.83-3.95 (m, 2 H), 4.08-4.14 (m, 2 H), 4.15-4.22 (m, 1 H), 7.93 (d, *J* = 1.8 Hz, 1 H), 8.04 (d, *J* = 2.0 Hz, 1 H), 8.07 (d, *J* = 2.0 Hz. 1 H), 8.89 (d, *J* = 2.3 Hz, 1 H).

### Example 65

### 4-[5-6-Diethylamino-5-methyl-pyridin-3-yl)-[1,3,4]oxadiazol-2-yl]-2-ethyl-6-methyl-phenol

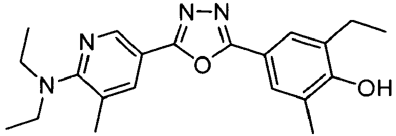

a) To a solution of 6-diethylamino-5-methyl-nicotinic acid (420 mg, 1.72 mmol) and DIPEA (665 mg, 5.15 mmol, 881 µL) in DMF (12 mL) PyBOP (999 mg, 1.92 mmol) is added at 0°C. The mixture is stirred at 0°C for 15 min before 4-benzyloxy-3-ethyl-5-methyl-benzoic acid hydrazide (1040 mg, 1.92 mmol) is added. Stirring is continued at 0°C for 4 h, then at rt for 16 h. The reaction is quenched with water (2 mL) and extracted with diethyl ether. The organic extract is washed with sat. aq. NaHCO₃ solution, dried over MgSO₄, filtered, and concentrated. The material is dissolved in DCM (60 mL) and pyridine (678 mg, 8.58 mmol) followed by trifluoromethanesulfonic acid anhydride (726 mg, 2.57 mmol) is added at 0°C. The mixture is stirred and warmed to rt overnight. The mixture is washed with 1 N aq. HCl and water, dried over MgSO₄, filtered and concentrated. The crude product is purified by CC eluting with heptane:EA 9:1 to give {5-[5-(4-benzyloxy-3,5-dimethyl-phenyl-[1,3,4]oxadiazol-2-yl]-3-methyl-pyridin-2-yl}-diethyl-amine (217 mg) as a colourless oil; LC-MS: t_{R} = 0.87 min; [M+1]⁺ = 457.23.
b) To a solution of the above benzyl ether (217 mg, 475 µmol) in THF (5 mL) and ethanol (5 mL), Pd/C (50 mg, 10% Pd) is carefully added. The slurry is stirred at rt for 15 h under 5 bar of H₂. The catalyst is removed by filtration and the filtrate is concentrated and dried to give the title compound (160 mg) as a white solid; LC-MS: t_{R} = 0.67 min; [M+1]⁺ =367.24.

### Example 66

### (S)-3-{4-[5-(6-Diethylamino-5-methyl-pyridin-3-yl)-[1,3,4]oxadiazol-2-yl]-2-ethyl-6-methyl-phenoxy}-propane-1,2-diol

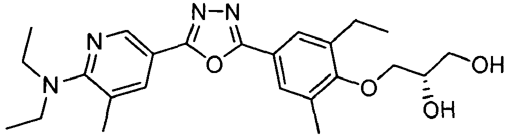

The title compound (51 mg) is prepared in analogy to Example 3 starting from Example 65 (50 mg, 136 µmol); LC-MS**: t_{R} = 0.60 min; [M+1]⁺ = 441.20;¹H NMR (CDCl₃): *δ*1.20 (t, *J* = 6.8 Hz, 6 H), 1.32 (t, *J* = 7.5 Hz, 3 H), 2.17 (t br, *J* = 5.8 Hz, 1 H), 2.38 (s, 3 H), 2.41 (s, 3 H), 2.77 (q, *J* = 7.5 Hz, 2 H), 3.44 (q, *J* = 7.3 Hz, 4 H), 3.58-3.76 (m, 2 H), 3.82-3.98 (m, 2 H), 4.15-4.21 (m, 1 H), 7.82 (s, 1 H), 7.83 (s, 1 H), 8.05 (d, *J* = 1.5 Hz, 1 H), 8.82 (d, *J* = 2.3 Hz, 1 H).

### Example 67

### 1-Amino-3-{4-[5-(6-diethylamino-5-methyl-pyridin-3-yl)-[1,3,4]oxadiazol-2-yl]-2-ethyl-6-methyl-phenoxy}-propan-2-ol

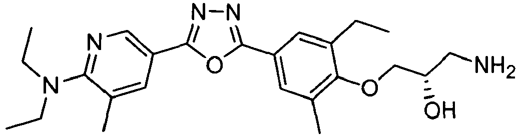

The title compound is prepared in analogy to Example 5 starting from Example 65; LC-MS**: t_{R} = 0.51 min; [M+1]⁺ = 440.27; ¹H NMR (CDCl₃): *δ*1.20 (t, *J* = 7.0 Hz, 6 H), 1.32 (t, *J* = 7.5 Hz, 3 H), 2.38 (s, 3 H), 2.41 (s, 3 H), 2.77 (q, *J* = 7.5 Hz, 2 H), 2.95 (dd, *J* = 12.8, 7.3 Hz, 1 H), 3.06 (dd, *J* = 12.8, 4.0 Hz, 1 H), 3.43 (q, *J* = 7.0 Hz, 4 H), 3.87-3.90 (m, 2 H), 4.01-4.06 (m, 1 H), 7.81 (s, 1 H), 7.83 (s, 1 H), 8.05 (d, *J* = 2.0 Hz, 1 H), 8.82 (d, *J* = 2.3 Hz, 1 H).

### Example 68

### N-((2S)-3-{4-[5-(6-Diethylamino-5-methyl-pyridin-3-yl)-[1,3,4]oxadiazol-2-yl]-2-ethyl-6-methyl-phenoxy}-2-hydroxy-propyl)-2-hydroxy-acetamide

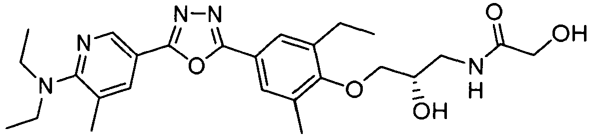

The title compound (16 mg) is prepared in analogy to Example 7 starting from Example 67 (15 mg, 33 µmol); LC-MS**: t_{R} = 0.57 min; [M+1]⁺ = 497.88; ¹H NMR (CDCl₃): *δ*1.21 (t, *J* = 7.0 Hz, 6 H), 1.31 (t, *J* = 7.5 Hz, 3 H), 2.39 (s, 6 H), 2.71 (s br, 1 H), 2.75 (q, *J* = 7.5 Hz, 2 H), 3.41-3.49 (m, 5 H), 3.49-3.57 (m, 1 H), 3.78-3.88 (m, 2 H), 3.91 (dd, *J* = 9.5, 4.8 Hz, 1 H), 4.19-4.26 (m, 3 H), 7.04 (t, *J =* 6.0 Hz, 1 H), 7.81 (s, 1 H), 7.82 (s, 1 H), 8.05 (s, 1 H), 8.82 (s, 1 H).

### Example 69

### (S)-1-{4-[5-(6-Diethylamino-5-methyl-pyridin-3-yl)-[1,3,4]oxadiazol-2-yl]-2-ethyl-6-methyl-phenoxy}-3-methylamino-propan-2-ol

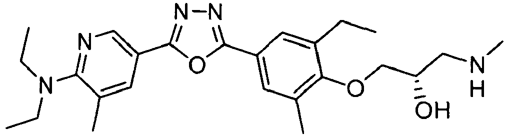

The title compound is prepared in analogy to Example 5 starting from Example 65 using methylamine in the epoxide opening step (step b); LC-MS**: t_{R} = 0.52 min; [M+1]⁺ = 454.39; ¹H NMR (CDCl₃): *δ*1.20 (t, *J* = 7.0 Hz, 6 H), 1.32 (t, *J* = 7.5 Hz, 3 H), 2.08 (s br, 2 H), 2.38 (s, 3 H), 2.41 (s, 3 H), 2.54 (s, 3 H), 2.78 (q, *J* = 7.5 Hz, 2 H), 2.81-2.91 (m, 2 H), 3.43 (q, *J* = 7.0 Hz, 4 H), 3.89 (d, *J =* 5.0 Hz, 2 H), 4.11-4.17 (m, 1 H), 7.81 (s, 1 H), 7.83 (s, 1 H), 8.05 (d, *J* = 1.8 Hz, 1 H), 8.82 (d, *J* = 2.3 Hz, 1 H).

### Example 70

### N-((2S)-3-{4-[5-(6-Diethylamino-5-methyl-pyridin-3-yl)-[1,3,4]oxadiazol-2-yl]-2-ethyl-6-methyl-phenoxy}-2-hydroxy-propyl)-2-hydroxy-N-methyl-acetamide

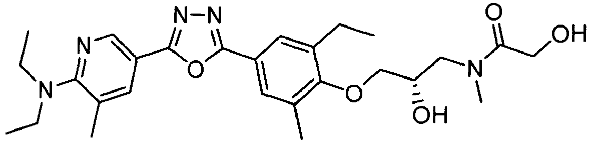

The title compound is prepared in analogy to Example 7 starting from Example 69; LC-MS**: t_{R}=0.61 min; [M+1]⁺ = 512.31 ; ¹H NMR (CDCl₃): *δ*1.21 (t, *J* = 7.0 Hz, 6 H), 1.32 (t, *J* = 7.5 Hz, 3 H), 2.38 (s, 3 H), 2.40 (s, 3 H), 2.76 (q, *J* = 7.5 Hz, 2 H), 3.10 (s, 3 H), 3.22 (d, *J* = 4.5 Hz, 1 H), 3.44 (q, *J* = 6.8 Hz, 4 H), 3.51 (t, *J* = 4.5 Hz, 1 H), 3.74-3.78 (m, 2 H), 3.80-3.88 (m, 1 H), 3.91 (dd, *J* = 9.5, 4.5 Hz, 1 H), 4.24-4.27 (m, 2 H), 4.29-4.37 (m, 1 H), 7.82 (s, 1 H), 7.84 (s, 1 H), 8.05 (d, *J* = 0.8 Hz, 1 H), 8.82 (d, *J* = 2.0 Hz, 1 H).

### Example 71: GTPγS assay to determine EC₅₀ values

GTPγS binding assays are performed in 96 well microtiter plates (Nunc, 442587) in a final volume of 200 µl, using membrane preparations of CHO cells expressing recombinant human S1 P1 receptor. Assay conditions are 20 mM Hepes (Fluka, 54461), 100 mM NaCl (Fluka, 71378), 5 mM MgCl₂ (Fluka, 63064), 0.1% BSA (Calbiochem, 126609), 1 µM GDP (Sigma, G-7127), 2.5% DMSO (Fluka, 41644), 50 pM ³⁵S-GTPγS (Amersham Biosciences, SJ1320). The pH is 7.4. Test compounds are dissolved and diluted in 100% DMSO and preincubated at room temperature for 30 min in 150 µl of the above assay buffer, in the absence of ³⁵S-GTPγS. After addition of 50 µl of ³⁵S-GTPγS, the assay is incubated for 1 h at rt. The assay is terminated by transfer of the reaction mixture to a Multiscreen plate (Millipore, MAHFC1H60) using a cell harvester from Packard Biosciences, and the plates are washed with ice-cold 10 mM Na₂HPO₄/NaH₂PO₄ (70%/30%), dried, sealed at the bottom and, after addition of 25 µl MicroScint20 (Packard Biosciences, order# 6013621), sealed on the top. Membrane-bound ³⁵S-GTPγS is measured with a TopCount from Packard Biosciences.

EC₅₀ is the concentration of agonist inducing 50 % of the maximal specific ³⁵S-GTPγS binding. Specific binding is determined by subtracting non-specific binding from maximal binding. Maximal binding is the amount of cpm bound to the Multiscreen plate in the presence of 10 µM of S1 P. Non-specific binding is the amount of binding in the absence of an agonist in the assay.

The EC₅₀ values of the compounds of Example 4, 5, 9 and 36 have not been measured. The EC₅₀ values of the compounds of Examples 18 and 19 have been measured greater than 10 µM. The EC₅₀ values of all other exemplified compounds are in the range of 0.2 nM to 6750 nM with an average of 571 nM. Agonistic activities, determined according to the method described above, of some compounds of the present invention are displayed in Table 1.

**Table 1:**

| **Compound of Example** | **EC₅₀ [nM]** |
|---|---|
| 6 | 3 |
| 15 | 20 |
| 24 | 12 |
| 27 | 8 |
| 28 | 2 |
| 31 | 17 |
| 34 | 3 |
| 44 | 4 |
| 46 | 5 |
| 54 | 5 |
| 55 | 0.9 |
| 56 | 1.3 |
| 58 | 14 |
| 63 | 5.2 |
| 64 | 8.9 |
| 68 | 21.2 |

### Example 72: Assessment of In vivo Efficacy

The efficacy of the compounds of Formula (I) is assessed by measuring the circulating lymphocytes after oral administration of 3 to 30 mg/kg of a compound of Formula (I) to normotensive male Wistar rats. The animals are housed in climate-controlled conditions with a 12 h-light/dark cycle, and have free access to normal rat chow and drinking water. Blood is collected before and 3, 6 and 24 h after drug administration. Full blood is subjected to hematology using Advia Hematology system (Bayer Diagnostics, Zürich, Switzerland).

All data are presented as mean ± SEM. Statistical analyses are performed by analysis of variance (ANOVA) using Statistica (StatSoft) and the Student-Newman-Keuls procedure for multiple comparisons. The null hypothesis is rejected when p < 0.05.

As an example, Table 2 shows the effect on lymphocyte counts 6 h after oral administration of 10 mg/kg of compounds of the present invention to normotensive male Wistar rats as compared to a group of animals treated with vehicle only.

**Table 2:**

| **Compound of Example** | **Lymphocyte counts** |
|---|---|
| 8 | -66 % |
| 27 | -55 % |
| 28 | -56 % |
| 54 | -57% |

## Claims

1. A compound of the Formula (I) 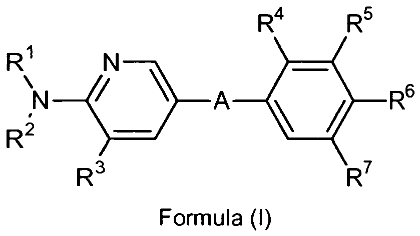 wherein
**A** represents 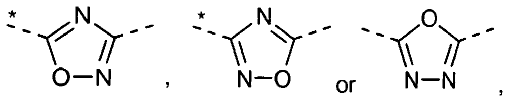 wherein the asterisks indicate the bond that is linked to the pyridine group of Formula (I);
**R**¹ represents C₁₋₄-alkyl;
**R²** represents hydrogen or C₁₋₃-alkyl;
or **R¹** and **R²**, together with the nitrogen to which they are attached, form an azetidine, or a pyrrolidine ring;
**R³** represents C₁₋₃-alkyl;
**R⁴** represents hydrogen, methoxy, or methyl;
**R⁵** represents hydrogen, C₁₋₃-alkyl, or methoxy;
**R⁶** represents -CH₂-(CH₂)ₙ-NR⁶¹R⁶², -CH₂-CH₂-CONR⁶¹R⁶², 1-(3-carboxy-azetidinyl-3-propionyl, 1-(2-carboxy-pyrrolidiny)-3-propionyl, 1-(3-carboxy-pyrrolidinyl)-3-propionyl, hydroxy, hydroxy-C₂₋₄-alkoxy, di-(hydroxy-C₁₋₂-alkyl)-C₁₋₂-alkoxy, 2,3-dihydroxypropoxy, -OCH₂-(CH₂)ₙ-NR⁶¹R⁶², -OCH₂-(CH₂)ₙ-NHCOR⁶³, 2-[(azetidine-3-carboxylic acid)-1-yl]-ethoxy, 2-[(pyrrolidine-2-carboxylic acid)-1-yl]-ethoxy, 2-[(pyrrolidine-3-carboxylic acid)-1-yl]-ethoxy, -OCH₂-CH(OH)-CH₂-NR⁶¹R⁶², -OCH₂-CH(OH)CH₂-NHCOR⁶³, 3-[(azetidine-3-carboxylic acid)-1-yl]-2-hydroxypropoxy, 2-hydroxy-3-[(pyrrolidine-2-carboxylic acid)-1-yl]-propoxy, or 2-hydroxy-3-[(pyrrolidine-3-carboxylic acid)-1-yl]-propoxy;
**R⁶¹** represents hydrogen, methyl, 2-hydroxyethyl, 2-aminoethyl, 2-(C₁₋₄-alkylamino)ethyl, 2-(di-(C₁₋₄-alkyl)amino)ethyl, carboxymethyl, (C₁₋₄-alkylcarboxy)methyl, 2-carboxyethyl, or 2-(C₁₋₄-alkylcarboxy)ethyl;
**R⁶²** represents hydrogen or methyl;
**R⁶³** represents hydroxymethyl, 2-hydroxyethyl, aminomethyl, methylaminomethyl, dimethylaminomethyl, or 2-methylamino-ethyl;
n represents the integer 1, or 2; and
**R⁷** represents hydrogen, methyl, or chloro;
or a salt of such a compound.

2. A compound according to claim 1, wherein **A** represents 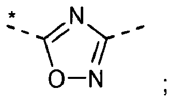 or a salt of such a compound.

3. A compound according to claims 1 or 2, wherein **R¹** represents C₁₋₃-alkyl;
or a salt of such a compound.

4. A compound according to any one of claims 1 to 3, wherein **R⁴** represents hydrogen;
or a salt of such a compound.

5. A compound according to any one of claims 1 to 4, wherein **R⁴** represents hydrogen,
**R⁵** represents ethyl, and **R⁷** represents methyl;
or a salt of such a compound.

6. A compound according to any one of claims 1 to 5, wherein **R⁶** represents -CH₂-CH₂-CONR⁶¹R⁶², 1-(3-carboxy-azetidinyl)-3-propionyl, 1-(2-carboxy-pyrrolidinyl)-3-propionyl, 1-(3-carboxy-pyrrolidinyl)-3-propionyl, hydroxy-C₂₋₄-alkoxy, di-(hydroxy-C₁₋₂-alkyl)-C₁₋₂-alkoxy, 2,3-dihydroxypropoxy, -OCH₂-(CH₂)ₙ-NR⁶¹R⁶², -OCH₂-(CH₂)ₙ-NHCOR⁶³, 2-[(azetidine-3-carboxylic acid)-1-yl]-ethoxy, 2-[(pyrrolidine-2-carboxylic acid)-1-yl]-ethoxy, 2-[(pyrrolidine-3-carboxylic acid)-1-yl]-ethoxy, -OCH₂-CH(OH)-CH₂-NR⁶¹-R⁶², -OCH₂-CH(OH)-CH₂-NHCOR⁶³, 3-[(azetidine-3-carboxylic acid)-1-yl]-2-hydroxypropoxy, 2-hydroxy-3-[(pyrrolidine-2-carboxylic acid)-yl]-propoxy, or 2-hydroxy-3-[(pyrrolidine-3-carboxylic acid)-1-y)]-propoxy;
or a salt of such a compound.

7. A compound according to any one of claims 1 to 6, wherein **R⁶** represents -CH₂-CH₂-CONR⁶¹R⁶², 2,3-dihydroxypropoxy, or -OCH₂-CH(OH)-CH₂-NHCOR⁶³; or a salt of such a compound.

8. A compound according to any one of claims 1 to 7, wherein
**R⁶¹** represents 2-hydroxyethyl, or carboxymethyl; **R⁶²** represents hydrogen; and **R⁶³** represents hydroxymethyl;
or a salt of such a compound.

9. A compound according to any one of claims 1 to 8, wherein
**R⁶** represents -OCH₂-CH(OH)-CH₂-NHCOR⁶³;
or a salt of such a compound.

10. A compound of Formula (I) according to claim 1 selected from the group consisting of:
N-[(R)-3-(2-Ethyl-4-{5-[6-(ethyl-methyl-amino)-5-methyl-pyridin-3-yl]-[1,2,4]oxadiazol-3-yl}-6-methyl-phenoxy)-2-hydroxy-propyl]-2-hydroxy-acetamide;
N-((S)-3-{4-[5-(6-Diethylamino-5-methyl-pyridin-3-yl)-[1,2,4]oxadiazol-3-yl]-2-ethyl-6-methyl-phenoxy}-2-hydroxy-propyl)-2-hydroxy-acetamide;
N-[(S)-3-(2-Ethyl-4-{5-[6-(isopropyl-methyl-amino)5-methyl-pyridin-3-yl]-[1,2,4]oxadiazol-3-yl)-6-methyl-phenoxy)-2-hydroxy-propyl]-2-hydroxy-acetamide;
(S)-3-{4-[5-(6-Diethylamino-5-ethyl-pyridin-3-yl)-[1,2,4]oxadiazol-3-yl]-2-ethyl-6-methyl-phenoxy}-propane-1,2-diol;
N-((R)-3-{4-[5-(6-Diethylamino-5-ethyl-pyridin-3-yl)-[1,2,4]oxadiazol-3-yl]-2-ethyl-6-methyl-phenoxy}-2-hydroxy-propyl)-2-hydroxy-acetamide;
N-((S)-3-{4-[5-(6-Diethylamino-5-ethyl-pyridin-3-yl)-[1,2,4]oxadiazol-3-yl]-2-ethyl-6-methyl-phenoxy}-2-hydroxy-propyl)-2-hydroxy-acetamide;
(S)-3-(2-Ethyl-4-{5-[5-ethyl-6-(isopropyl-methyl-amino)pyridin-3-yl]-[1,2,4]oxadiazol-3-yl}-6-methyl-phenoxy)-propane-1,2-diol;
N-[(R)-3-(2-Ethyl-4-{5-[5-ethyl-6-(isopropyl-methyl-amino)-pyridin-3-yl]-[1,2,4]oxadiazol-3-yl}-6-methyl-phenoxy)-2-hydroxy-propyl]-2-hydroxy-acetamide;
N-[(S)-3-(2-Ethyl-4-{5-[5-ethyl-6-(isopropyl-methyl-amino}-pyridin-3-yl]-[1,2,4]oxadiazol-3-yl}-6-methyl-phenoxyy2-hydroxy-propyl]-2-hydroxy-acetamide;
(3-{4-[5-(6-Diethylamino-5-ethyl-pyridin-3-yl)-[1,2,4]oxadiazol-3-yl]-2-ethyl-6-methyl-phenyl}-propionylamino)-acetic acid;
3-(2-Ethyl-4-{5-[5-ethyl-6-(isopropyl-methyl-amino)-pyridin-3-yl]-[1,2,4]oxadiazol-3-yl}-6-methyl-phenyl)-N-(2-hydroxy-ethyl)-propionamide; and
[3-(2-Ethyl-4-{5-[5-ethyl-6-(isopropyl-methyl-amino)-pyridin-3-yl]-[1,2,4]oxadiazol-3-yl}-6-methyl-phenyl)-propionylamino]-acetic acid;
or a salt of such a compound.

11. A compound of Formula (I) according to claim 1 selected from the group consisting of:
N-((S)-3-{2-Ethyl-4-[5-(5-ethy)-6-pyrrolidin-1-yl-pyridin-3-yl)-[1,2,4]oxadiazol-3-y)]-6-methyl-phenoxy}-2-hydroxy-propyl)2-hydroxy-acetamide;
N-((S)-3-{4-[5-(6-Diethylamino-5-methyl-pyridin-3-yl)-[1,2,4]oxadiazol-3-yl]-2,6-dimethy)-phenoxy)-2-hydroxy-propyl)-2-hydroxy-acetamide;
N-((S)-3-{2-Chloro-4-[5-(6-diethylamino-5-methyl-pyridin-3-yl)-[1,2,4]oxadiazol-3-yl]-6-methyl-phenoxy}-2-hydroxy-propyl)-2-hydroxy-acetamide;
N-((S)-3-{4-[5-(6-Diethylamino-5-methy)-pyridin-3-yl}-[1,2,4]oxadiazo)-3-yl]-2-methoxy-6-methyl-phenoxy}-2-hydroxy-propyl}-2-hydroxy-acetamide; N-((S)-3-{4-[5-(6-Diethylamino-5-methyl-pyridin-3-yl)-[1,2,4]oxadiazol-3-yl]-3-methyl-phenoxy}-2-hydroxy-propyl)-2-hydroxy-acetamide;
(S)-3-{2-Chloro-4-[5-(6-diethylamino-5-methyl-pyridin-3-yl)-[1,2,4]oxadiazol-3-yl]-6-methyl-phenoxy}-propane-1,2-diol; and (S)-3-{2-Chloro-4-[5-(6-diethylamino-5-methyl-pyridin-3-yl)-[1,2,4]oxadiazol-3-yl]-6-methoxy-phenoxy}-propane-1,2-diol;
or a salt of such a compound.

12. A pharmaceutical composition comprising a compound according to any one of claims 1 to 11, or a pharmaceutically acceptable salt thereof, and a pharmaceutically acceptable carrier.

13. A compound according to any one of claims 1 to 11, or a pharmaceutically acceptable salt thereof, or a pharmaceutical composition according to claim 12, for use as a medicament.

14. Use of a compound according to any one of claims 1 to 11, or of a pharmaceutically acceptable salt thereof, for the preparation of a pharmaceutical composition for the prevention or treatment of a disease or disorder associated with an activated immune system.

15. The use according to claim 14 for the prevention or treatment of a disease or disorder selected from the group consisting of rejection of transplanted organs such as kidney, liver, heart, lung, pancreas, cornea, and skin; graft-versus-host diseases brought about by stem cell transplantation; autoimmune syndromes including rheumatoid arthritis, multiple sclerosis, inflammatory bowel diseases such as Crohn's disease and ulcerative colitis, psoriasis, psoriatic arthritis, thyroiditis such as Hashimoto's thyroiditis, uveo-retinitis; atopic diseases such as rhinitis, conjunctivitis, dermatitis; asthma; type I diabetes; post-infectious autoimmune diseases including rheumatic fever and post-infectious glomerulonephritis; solid cancers and tumor metastasis.

16. A compound according to any one of claims 1 to 11, or a pharmaceutically acceptable salt thereof, for the prevention or treatment of a disease or disorder listed in claim 15.

## Patentansprüche

1. Verbindung der Formel (I) 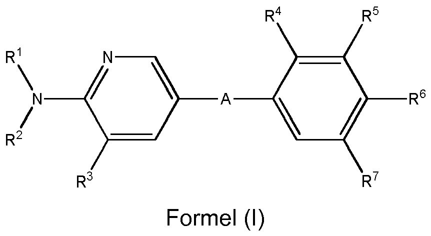 wobei
**A** 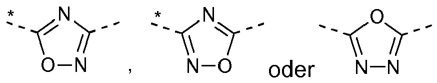 repräsentiert,
wobei die Sternchen die mit der Pyridingruppe der Formel (I) verknüpfte Bindung andeuten;
**R¹** C₁₋₄-Alkyl repräsentiert;
**R²** Wasserstoff oder C₁₋₃-Alkyl repräsentiert;
oder **R¹** und **R²** zusammen mit dem Stickstoff, an den sie gebunden sind, einen Azetidin- oder einen Pyrrolidinring bilden;
**R³** C₁₋₃-Alkyl repräsentiert;
**R⁴** Wasserstoff, Methoxy oder Methyl repräsentiert;
**R⁵** Wasserstoff, C₁₋₃-Alkyl oder Methoxy repräsentiert;
**R⁶** -CH₂-(CH₂)ₙ-NR⁶¹R⁶², -CH₂-CH₂-CONR⁶¹R⁶², 1-(3-Carboxy-azetidinyl)-3-propionyl, 1-(2-Carboxy-pyrrolidinyl)-3-propionyl, 1-(3-Carboxy-pyrrolidinyl)-3-propionyl, Hydroxy, Hydroxy-C₂₋₄-alkoxy, Di-(hydroxy-C₁₋₂-alkyl)-C₁₋₂-alkoxy, 2,3-Dihydroxypropoxy, -OCH₂-(CH₂)ₙ-NR⁶¹R⁶², -OCH₂-(CH₂)ₙ-NHCOR⁶³, 2-[(Azetidin-3-carbonsäure)-1-yl]-ethoxy, 2-[(Pyrrolidin-2-carbonsäure)-1-yl]-ethoxy, 2-[(Pyrrolidin-3-carbonsäure)-1-yl]-ethoxy, -OCH₂-CH(OH)-CH₂-NR⁶¹R⁶², -OCH₂-CH(OH)-CH₂-NHCOR⁶³, 3-[(Azetidin-3-carbonsäure)-1-yl]-2-hydroxypropoxy, 2-Hydroxy-3-[(pyrrolidin-2-carbonsäure)-1-yl]-propoxy oder 2-Hydroxy-3-[(pyrrolidin-3-carbonsäure)-1-yl]-propoxy repräsentiert;
**R⁶¹** Wasserstoff, Methyl, 2-Hydroxyethyl, 2-Aminoethyl, 2-(C₁₋₄-Alkylamino)ethyl, 2-(Di-(C₁₋₄-alkyl)amino)ethyl, Carboxymethyl, (C₁₋₄-Alkylcarboxy)methyl, 2-Carboxyethyl oder 2-(C₁₋₄-Alkylcarboxy)ethyl repräsentiert;
**R⁶²** Wasserstoff oder Methyl repräsentiert;
**R⁶³** Hydroxymethyl, 2-Hydroxyethyl, Aminomethyl, Methylaminomethyl, Dimethylaminomethyl oder 2-Methylamino-ethyl repräsentiert;
n die ganze Zahl 1 oder 2 repräsentiert; und
**R⁷** Wasserstoff, Methyl oder Chlor repräsentiert; oder ein Salz einer solchen Verbindung.

2. Verbindung nach Anspruch 1, wobei **A** 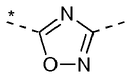 repräsentiert;
oder ein Salz einer solchen Verbindung.

3. Verbindung nach den Ansprüchen 1 oder 2, wobei **R¹** C₁₋₃-Alkyl repräsentiert; oder ein Salz einer solchen Verbindung.

4. Verbindung nach einem der Ansprüche 1 bis 3, wobei **R⁴** Wasserstoff repräsentiert; oder ein Salz einer solchen Verbindung.

5. Verbindung nach einem der Ansprüche 1 bis 4, wobei **R**⁴ Wasserstoff repräsentiert, **R⁵** Ethyl repräsentiert und **R⁷** Methyl repräsentiert;
oder ein Salz einer solchen Verbindung.

6. Verbindung nach einem der Ansprüche 1 bis 5, wobei **R⁶** Folgendes repräsentiert:
-CH₂-CH₂-CONR⁶¹R⁶², 1-(3-Carboxy-azetidinyl)-3-propionyl, 1-(2-Carboxy-pyrrolidinyl)-3-propionyl, 1-(3-Carboxy-pyrrolidinyl)-3-propionyl, Hydroxy-C₂₋₄-alkoxy, Di-(hydroxy-C₁₋₂-alkyl)-C₁₋₂-alkoxy, 2,3-Dihydroxypropoxy, -OCH₂-(CH₂)ₙ-NR⁶¹R⁶², -OCH₂-(CH₂)ₙ-NHCOR⁶³, 2-[(Azetidin-3-carbonsäure)-1-yl]-ethoxy, 2-[(Pyrrolidin-2-carbonsäure)-1-yl]-ethoxy, 2-[(Pyrrolidin-3-carbonsäure)-1-yl]-ethoxy, -OCH₂-CH(OH)-CH₂-NR⁶¹R⁶², -OCH₂-CH(OH)-CH₂-NHCOR⁶³, 3-[(Azetidin-3-carbonsäure)-1-yl]-2-hydroxypropoxy, 2-Hydroxy-3-[(pyrrolidin-2-carbonsäure)-1-yl]-propoxy oder 2-Hydroxy-3-[(pyrrolidin-3-carbonsäure)-1-yl]-propoxy; oder ein Salz einer solchen Verbindung.

7. Verbindung nach einem der Ansprüche 1 bis 6, wobei **R⁶**
-CH₂-CH₂-CONR⁶¹R⁶², 2,3-Dihydroxypropoxy oder -OCH₂-CH(OH)-CH₂-NHCOR⁶³ repräsentiert;
oder ein Salz einer solchen Verbindung.

8. Verbindung nach einem der Ansprüche 1 bis 7, wobei
**R⁶¹** 2-Hydroxyethyl oder Carboxymethyl repräsentiert; **R⁶²** Wasserstoff repräsentiert; und **R⁶³** Hydroxymethyl repräsentiert;
oder ein Salz einer solchen Verbindung.

9. Verbindung nach einem der Ansprüche 1 bis 8, wobei
**R⁶** -OCH₂-CH(OH)-CH₂-NHCOR⁶³ repräsentiert;
oder ein Salz einer solchen Verbindung.

10. Verbindung der Formel (I) nach Anspruch 1, die ausgewählt ist aus der Gruppe bestehend aus:
N-[(R)-3-(2-Ethyl-4-{5-[6-(ethyl-methyl-amino)-5-methyl-pyridin-3-yl]-[1,2,4]oxadiazol-3-yl}-6-methyl-phenoxy)-2-hydroxy-propyl]-2-hydroxy-acetamid;
N-((S)-3-(4-[5-(6-Diethylamino-5-methyl-pyridin-3-yl)-[1,2,4]oxadiazol-3-yl]-2-ethyl-6-methyl-phenoxy}-2-hydroxy-propyl)-2-hydroxy-acetamid;
N-[(S)-3-(2-Ethyl-4-{5-[6-(isopropyl-methyl-amino)-5-methyl-pyridin-3-yl]-[1,2,4]oxadiazol-3-yl}-6-methyl-phenoxy)-2-hydroxy-propyl]-2-hydroxy-acetamid;
(S)-3-(4-[5-(6-Diethylamino-5-ethyl-pyridin-3-yl)-[1,2,4]oxadiazol-3-yl]-2-ethyl-6-methyl-phenoxy}-propan-1,2-diol;
N-((R)-3-(4-[5-(6-Diethylamino-5-ethyl-pyridin-3-yl)-[1,2,4]oxadiazol-3-yl]-2-ethyl-6-methyl-phenoxy}-2-hydroxy-propyl)-2-hydroxy-acetamid;
N-((S)-3-{4-[5-(6-Diethylamino-5-ethyl-pyridin-3-yl)-[1,2,4]oxadiazol-3-yl]-2-ethyl-6-methyl-phenoxy}-2-hydroxy-propyl)-2-hydroxy-acetamid;
(S)-3-(2-Ethyl-4-{5-[5-ethyl-6-(isopropyl-methyl-amino)-pyridin-3-yl]-[1,2,4]oxadiazol-3-yl}-6-methyl-phenoxy)-propan-1,2-diol;
N-[(R)-3-(2-Ethyl-4-{5-[5-ethyl-6-(isopropyl-methyl-amino)-pyridin-3-yl]-[1,2,4]oxadiazol-3-yl}-6-methyl-phenoxy)-2-hydroxy-propyl]-2-hydroxy-acetamid;
N-[(S)-3-(2-Ethyl-4-{5-[5-ethyl-6-(isopropyl-methyl-amino)-pyridin-3-yl]-[1,2,4]oxadiazol-3-yl}-6-methyl-phenoxy)-2-hydroxy-propyl]-2-hydroxy-acetamid;
(3-{4-[5-(6-Diethylamino-5-ethyl-pyridin-3-yl)-[1,2,4]oxadiazol-3-yl]-2-ethyl-6-methyl-phenyl}-propionylamino)-essigsäure;
3-(2-Ethyl-4-{5-[5-ethyl-6-(isopropyl-methyl-amino)-pyridin-3-yl]-[1,2,4]oxadiazol-3-yl}-6-methyl-phenyl)-N-(2-hydroxy-ethyl)-propionamid; und
[3-(2-Ethyl-4-{5-[5-ethyl-6-(isopropyl-methyl-amino)-pyridin-3-yl]-[1,2,4]oxadiazol-3-yl}-6-methyl-phenyl)-propionylamino]-essigsäure;
oder ein Salz einer solchen Verbindung.

11. Verbindung der Formel (I) nach Anspruch 1, die ausgewählt ist aus der Gruppe bestehend aus:
N-((S)-3-{2-Ethyl-4-[5-(5-ethyl-6-pyrrolidin-1-yl-pyridin-3-yl)-[1,2,4]oxadiazol-3-yl]-6-methyl-phenoxy}-2-hydroxy-propyl)-2-hydroxy-acetamid;
N-((S)-3-{4-[5-(6-Diethylamino-5-methyl-pyridin-3-yl)-[1,2,4]oxadiazol-3-yl]-2,6-dimethyl-phenoxy}-2-hydroxy-propyl)-2-hydroxy-acetamid;
N-((S)-3-{2-Chlor-4-[5-(6-diethylamino-5-methyl-pyridin-3-yl)-[1,2,4]oxadiazol-3-yl]-6-methyl-phenoxy}-2-hydroxy-propyl)-2-hydroxy-acetamid;
N-((S)-3-{4-[5-(6-Diethylamino-5-methyl-pyridin-3-yl)-[1,2,4]oxadiazol-3-yl]-2-methoxy-6-methyl-phenoxy}-2-hydroxy-propyl)-2-hydroxy-acetamid;
N-((S)-3-{4-[5-(6-Diethylamino-5-methyl-pyridin-3-yl)-[1,2,4]oxadiazol-3-yl]-3-methyl-phenoxy}-2-hydroxy-propyl)-2-hydroxy-acetamid;
(S)-3-{2-Chlor-4-[5-(6-diethylamino-5-methyl-pyridin-3-yl)-[1,2,4]oxadiazol-3-yl]-6-methyl-phenoxy}-propan-1,2-diol; und
(S)-3-{2-Chlor-4-[5-(6-diethylamino-5-methyl-pyridin-3-yl)-[1,2,4]oxadiazo1-3-yl]-6-methoxy-phenoxy}-propan-1,2-diol;
oder ein Salz einer solchen Verbindung.

12. Pharmazeutische Zusammensetzung, die eine Verbindung nach einem der Ansprüche 1 bis 11 oder ein pharmazeutisch akzeptables Salz davon und einen pharmazeutisch akzeptablen Träger umfasst.

13. Verbindung nach einem der Ansprüche 1 bis 11 oder ein pharmazeutisch akzeptables Salz davon oder eine pharmazeutische Zusammensetzung nach Anspruch 12 zur Verwendung als Medikament.

14. Verwendung einer Verbindung nach einem der Ansprüche 1 bis 11 oder eines pharmazeutisch akzeptablen Salzes davon zur Herstellung einer pharmazeutischen Zusammensetzung zur Verhütung oder Behandlung einer Krankheit oder Störung, die mit einem aktivierten Immunsystem assoziiert ist.

15. Verwendung nach Anspruch 14 zur Verhütung oder Behandlung einer Krankheit oder Störung, die ausgewählt ist aus der Gruppe bestehend aus der Abstoßung transplantierter Organe wie Niere, Leber, Herz, Lunge, Bauchspeicheldrüse, Hornhaut und Haut; Transplantat-gegen-Empfänger-Krankheiten infolge einer Stammzellentransplantation; Autoimmunsyndromen wie Rheumatoidarthritis, Multiple Sklerose, entzündliche Darmerkrankungen wie die Crohn-Krankheit und Colitis ulcerosa, Psoriasis, psoriatische Arthritis, Thyreoiditis wie Hashimoto-Thyreoiditis, Uveoretinitis; atopischen Krankheiten wie Rhinitis, Konjunktivitis, Dermatitis; Asthma; Typ-I-Diabetes; postinfektiösen Autoimmunkrankheiten wie rheumatisches Fieber und postinfektiöse Glomerulonephritis; soliden Krebsarten und Tumormetastasen.

16. Verbindung nach einem der Ansprüche 1 bis 11 oder ein pharmazeutisch akzeptables Salz davon zur Verhütung oder Behandlung einer in Anspruch 15 aufgeführten Krankheit oder Störung.

## Revendications

1. Composé de formule (I) 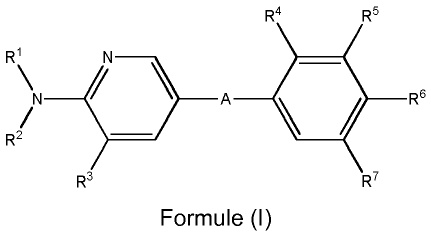 où A représente 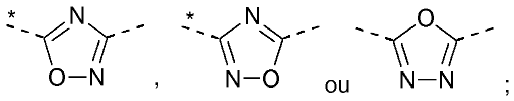 où les astérisques indiquent la liaison avec le groupement pyridine de formule (I) ;
**R¹** représente un C₁₋₄-alkyle ;
**R²** représente un hydrogène ou un C₁₋₃-alkyle ;
ou **R¹** et **R²,** avec l'atome d'azote qui les porte, forment un noyau azétidine ou pyrrolidine ; **R³** représente un C₁₋₃-alkyle ;
**R⁴** représente un hydrogène, un méthoxy ou un méthyle ;
**R⁵** représente un hydrogène, un C₁₋₃-alkyle ou un méthoxy ;
**R⁶** représente un groupement -CH₂-(CH₂)ₙ-NR⁶¹R⁶², -CH₂-CH₂-CONR⁶¹R⁶², 1-(3-carboxy-azétidinyl)-3-propionyle, 1-(2-carboxy-pyrrolidinyl)-3-propionyle, 1-(3-carboxy-pyrrolidinyl)-3-propionyle, hydroxy, hydroxy-C₂₋₄-alkoxy, di-(hydroxy-C₁₋₂-alkyle)-C₁₋₂-alkoxy, 2,3-dihydroxypropoxy, -OCH₂-(CH₂)ₙ-NR⁶¹R⁶², -OCH₂-(CH₂)ₙ-NHCOR⁶³, 2-[(acide azétidine-3-carboxylique)-1-yl]-éthoxy, 2-[(acide pyrrolidine-2-carboxylique)-1-yl]-éthoxy, 2-[(acide pyrrolidine-3-carboxylique)-1-yl]-éthoxy, -OCH₂-CH(OH)-CH₂-NR⁶¹R⁶², -OCH₂-CH(OH)-CH₂-NHCOR⁶³, 3-[(acide azétidine-3-carboxylique)-1-yl]-2-hydroxypropoxy, 2-hydroxy-3-[(acide pyrrolidine-2-carboxylique)-1-yl]-propoxy ou 2-hydroxy-3-[(acide pyrrolidine-3-carboxylique)-1-yl]-propoxy ;
**R⁶¹** représente un hydrogène, méthyle, 2-hydroxyéthyle, 2-aminoéthyle, 2-(C₁₋₄-alkylamino)éthyle, 2-(di-(C₁₋₄-alkyl)amino)éthyle, carboxyméthyle, (C₁₋₄-alkylcarboxy)méthyle, 2-carboxyéthyle ou 2-(C₁₋₄-alkylcarboxy)éthyle ;
**R⁶²** représente un hydrogène ou un méthyle ;
**R⁶³** représente un hydroxyméthyle, 2-hydroxyéthyle, aminométhyle, méthylaminométhyle, diméthylaminométhyle ou 2-méthylamino-éthyle ;
n représente le nombre entier 1 ou 2 ; et
**R⁷** représente un hydrogène, un méthyle ou un chloro ;
ou sel d'un tel composé.

2. Composé selon la revendication 1, où **A** représente 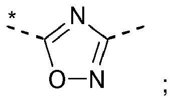 ou sel d'un tel composé.

3. Composé selon la revendication 1 ou 2, où **R¹** représente un C₁₋₃-alkyle ;
ou sel d'un tel composé.

4. Composé selon l'une quelconque des revendications 1 à 3, où **R⁴** représente un hydrogène ;
ou sel d'un tel composé.

5. Composé selon l'une quelconque des revendications 1 à 4, où **R⁴** représente un hydrogène, **R⁵** représente un éthyle et **R⁷** représente un méthyle ;
ou sel d'un tel composé.

6. Composé selon l'une quelconque des revendications 1 à 5, où **R⁶** représente un groupement -CH₂-CH₂-CONR⁶¹R⁶², 1-(3-carboxy-azétidinyl)-3-propionyle, 1-(2-carboxy-pyrrolidinyl)-3-propionyle, 1-(3-carboxy-pyrrolidinyl)-3-propionyle, hydroxy-C₂₋₄-alkoxy, di-(hydroxy-C₁₋₂-alkyle)-C₁₋₂-alkoxy, 2,3-dihydroxypropoxy, -OCH₂-(CH₂)ₙ-NR⁶¹R⁶², -OCH₂-(CH₂)ₙ-NHCOR⁶³, 2-[(acide azétidine-3-carboxylique)-1-yl]-éthoxy, 2-[(acide pyrrolidine-2-carboxylique)-1-yl]-éthoxy, 2-[(acide pyrrolidine-3-carboxylique)-1-yl]-éthoxy, -OCH₂-CH(OH)-CH₂-NR⁶¹R⁶², -OCH₂-CH(OH)-CH₂-NHCOR⁶³, 3-[(acide azétidine-3-carboxylique)-1-yl]-2-hydroxypropoxy, 2-hydroxy-3-[(acide pyrrolidine-2-carboxylique)-1-yl]-propoxy ou 2-hydroxy-3-[(acide pyrrolidine-3-carboxylique)-1-yl]-propoxy ;
ou sel d'un tel composé.

7. Composé selon l'une quelconque des revendications 1 à 6, où **R⁶** représente un groupement -CH₂-CH₂-CONR⁶¹R⁶², 2,3-dihydroxypropoxy ou -OCH₂-CH(OH)-CH₂-NHCOR⁶³ _{;}
ou sel d'un tel composé.

8. Composé selon l'une quelconque des revendications 1 à 7, où **R⁶¹** représente un 2-hydroxyéthyle ou un carboxyméthyle ; **R⁶²** représente un hydrogène ; et **R⁶³** représente un hydroxyméthyle ;
ou sel d'un tel composé.

9. Composé selon l'une quelconque des revendications 1 à 8, où **R⁶** représente un groupement -OCH₂-CH(OH)-CH₂-NHCOR⁶³ ;
ou sel d'un tel composé.

10. Composé de formule (I) selon la revendication 1, sélectionné dans le groupe consistant en les suivants :
N-[(R)-3-(2-Éthyl-4-{5-[6-(éthyl-méthyl-amino)-5-méthyl-pyridin-3-yl]-[1,2,4]oxadiazol-3-yl}-6-méthyl-phénoxy)-2-hydroxy-propyl]-2-hydroxy-acétamide;
N-((S)-3-{4-[5-(6-Diéthylamino-5-méthyl-pyridin-3-yl)-[1,2,4]oxadiazol-3-yl]-2-éthyl-6-méthyl-phénoxy}-2-hydroxy-propyl)-2-hydroxy-acétamide ;
N-[(S)-3-(2-Éthyl-4-{5-[6-(isopropyl-méthyl-amino)-5-méthyl-pyridin-3-yl]-[1,2,4]oxadiazol-3-yl}-6-méthyl-phénoxy)-2-hydroxy-propyl]-2-hydroxy-acétamide;
(S)-3-{4-[5-(6-Diéthylamino-5-éthyl-pyridin-3-yl)-[1,2,4]oxadiazol-3-yl]-2-éthyl-6-méthyl-phénoxy}-propane-1,2-diol;
N-((R)-3-{4-[5-(6-Diéthylamino-5-éthyl-pyridin-3-yl)-[1,2,4]oxadiazol-3-yl]-2-éthyl-6-méthyl-phénoxy}-2-hydroxy-propyl)-2-hydroxy-acétamide ;
N-((S)-3-{4-[5-(6-Diéthylamino-5-éthyl-pyridin-3-yl)-[1,2,4]oxadiazol-3-yl]-2-éthyl-6-méthyl-phénoxy}-2-hydroxy-propyl)-2-hydroxy-acétamide ;
(S)-3-(2-Éthyl-4-{5-[5-éthyl-6-(isopropyl-méthyl-amino)-pyridin-3-yl]-[1,2,4]oxadiazol-3-yl}-6-méthyl-phénoxy)-propane-1,2-diol ;
N-[(R)-3-(2-Éthyl-4-{5-[5-éthyl-6-(isopropyl-méthyl-amino)-pyridin-3-yl]-[1,2,4]oxadiazol-3-yl}-6-méthyl-phénoxy)-2-hydroxy-propyl]-2-hydroxy-acétamide;
N-[(S)-3-(2-Éthyl-4-{5-[5-éthyl-6-(isopropyl-méthyl-amino)-pyridin-3-yl]-[1,2,4]oxadiazol-3-yl}-6-méthyl-phénoxy)-2-hydroxy-propyl]-2-hydroxy-acétamide;
Acide (3-{4-[5-(6-diéthylamino-5-éthyl-pyridin-3-yl)-[1,2,4]oxadiazol-3-yl]-2-éthyl-6-méthyl-phényl}-propionylamino)-acétique ;
3-(2-Éthyl-4-{5-[5-éthyl-6-(isopropyl-méthyl-amino)-pyridin-3-yl]-[1,2,4]oxadiazol-3-yl}-6-méthyl-phényl)-N-(2-hydroxy-éthyl)-propionamide ; et
Acide [3-(2-éthyl-4-{5-[5-éthyl-6-(isopropyl-méthyl-amino)-pyridin-3-yl]-[1,2,4]oxadiazol-3-yl}-6-méthyl-phényl)-propionylamino]-acétique;
ou sel d'un tel composé.

11. Composé de formule (I) selon la revendication 1, sélectionné dans le groupe consistant en les suivants :
N-((S)-3-{2-Éthyl-4-[5-(5-éthyl-6-pyrrolidin-1-yl-pyridin-3-yl)-[1,2,4]oxadiazol-3-yl]-6-méthyl-phénoxy}-2-hydroxy-propyl)-2-hydroxy-acétamide ;
N-((S)-3-{4-[5-(6-Diéthylamino-5-méthyl-pyridin-3-yl)-[1,2,4]oxadiazol-3-yl]-2,6-diméthyl-phénoxy}-2-hydroxy-propyl)-2-hydroxy-acétamide ;
N-((S)-3-{2-Chloro-4-[5-(6-diéthylamino-5-méthyl-pyridin-3-yl)-[1,2,4]oxadiazol-3-yl]-6-méthyl-phénoxy}-2-hydroxy-propyl)-2-hydroxy-acétamide;
N-((S)-3-{4-[5-(6-Diéthylamino-5-méthyl-pyridin-3-yl)-[1,2,4]oxadiazol-3-yl]-2-méthoxy-6-méthyl-phénoxy}-2-hydroxy-propyl)-2-hydroxy-acétamide; N-((S)-3-{4-[5-(6-Diéthylamino-5-méthyl-pyridin-3-yl)-[1,2,4]oxadiazol-3-yl]-3-méthyl-phénoxy}-2-hydroxy-propyl)-2-hydroxy-acétamide ;
(S)-3-{2-Chloro-4-[5-(6-diéthylamino-5-méthyl-pyridin-3-yl)-[1,2,4]oxadiazol-3-yl]-6-méthyl-phénoxy}-propane-1,2-diol ; et
(S)-3-{2-Chloro-4-[5-(6-diéthylamino-5-méthyl-pyridin-3-yl)-[1,2,4]oxadiazol-3-yl]-6-méthoxy-phénoxy}-propane-1,2-diol ;
ou sel d'un tel composé.

12. Composition pharmaceutique qui comprend un composé selon l'une quelconque des revendications 1 à 11 ou un sel pharmaceutiquement acceptable de celui-ci, et un véhicule pharmaceutiquement acceptable.

13. Composé selon l'une quelconque des revendications 1 à 11 ou sel pharmaceutiquement acceptable de celui-ci, ou composition pharmaceutique selon la revendication 12, en vue d'une utilisation en tant que médicament.

14. Utilisation d'un composé selon l'une quelconque des revendications 1 à 11 ou d'un sel pharmaceutiquement acceptable de celui-ci dans la préparation d'une composition pharmaceutique indiquée dans la prévention ou le traitement d'une maladie ou d'une affection associée à un système immunitaire activé.

15. Utilisation selon la revendication 14 dans la prévention ou le traitement d'une maladie ou affection sélectionnée dans le groupe consistant en les suivantes : rejet d'organes transplantés, par exemple rein, foie, coeur, poumon, pancréas, cornée et peau ; maladies du greffon contre l'hôte déclenchées par une greffe de cellules souches ; syndromes auto-immuns, y compris polyarthrite rhumatoïde, sclérose en plaques, affections intestinales inflammatoires comme la maladie de Crohn et la rectocolite hémorragique, psoriasis, rhumatisme psoriasique, thyroïdite, comme la thyroïdite chronique de Hashimoto, uvéorétinite ; maladies atopiques comme la rhinite, conjonctivite, dermatite ; asthme ; diabète de type I ; maladies auto-immunes post-infectieuses, y compris rhumatisme articulaire aigu et glomérulonéphrite post-infectieuse ; cancers solides et métastases tumorales.

16. Composé selon l'une quelconque des revendications 1 à 11 ou sel pharmaceutiquement acceptable de celui-ci, pour la prévention ou le traitement d'une maladie ou affection répertoriée à la revendication 15.
